# EUROPEAN PATENT APPLICATION

(11) **EP 3 998 282 A1**
(43) Date of publication of application: **18.05.2022**
(21) Application number: 20836293.9
(22) Date of filing: 07.07.2020
(51) Int. Cl.: C07K 16/28, C07K 14/54, A61K 38/20, A61K 47/68, A61P 37/00

(54) **NOVEL FUSION PROTEIN AND USE OF SAME**

(30) Priority: 08.07.2019 KR 20190082151
(71) Applicant: Progen Co., Ltd., Seoul 06591 (KR)
(72) Inventor: JIN, Hyuntak, Seongnam-si Gyeonggi-do 13556 (KR); CHOI, Bokyung, Suwon-si Gyeonggi-do 16226 (KR)
(74) Representative: Dehns
(86) International application number: PCT/KR2020/008870
(87) International publication number: WO 2021/006604

(57) **Abstract**

The present invention relates to a novel fusion protein and use thereof, and more particularly, to a fusion protein specifically binds to CD154 comprising Fab or scFv specifically binding to CD154 Fc regions, wherein the Fc region is a modified Fc region which is modified so as not to bind to an Fc gamma receptor.

<110>    PROGEN CO., LTD.

<120>    A novel fusion protein and use thereof

<130>    PT20-5201

<150>    KR 10-2019-0082151
<151>    2019-07-08

<160>    49

<170>    KoPatentIn 3.0

<210>    1
<211>    446
<212>    PRT
<213>    Artificial Sequence

<220>
<223>    chimeric anti-CD154 antibody heavy chain


<400>    1
Glu Val Gln Leu Gln Gln Ser Gly Ser Glu Leu Val Lys Pro Gly Ala
  1               5                  10                  15

Ser Val Lys Leu Ser Cys Lys Ala Ser Gly Tyr Ile Phe Thr Ser Tyr
             20                  25                  30

Tyr Met Tyr Trp Val Lys Gln Arg Pro Gly Gln Gly Leu Glu Trp Ile
         35                  40                  45

Gly Glu Ile Asn Pro Ser Asn Gly Asp Thr Asn Phe Asn Glu Lys Phe
     50                  55                  60

Lys Ser Lys Ala Thr Leu Thr Val Asp Lys Ser Ser Ser Thr Ala Tyr
 65                  70                  75                  80

Met Gln Leu Ile Ser Leu Thr Ser Glu Asp Ser Ala Val Tyr Tyr Cys
                 85                  90                  95

Thr Arg Ser Asp Gly Arg Asn Asp Met Asp Ser Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr

210                 215                 220

His Thr Cys Pro Pro Cys Pro Ser His Thr Gln Pro Leu Gly Val Phe
225                 230                 235                 240

Leu Phe Pro Pro Lys Pro Lys Asp Gln Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Leu His Glu Ala Leu His
            420                 425                 430

Asn His Tyr Thr Gln Lys Ser Leu Ser Leu Ser Leu Gly Lys
        435                 440                 445

<210>   2
<211>   218
<212>   PRT
<213>   Artificial Sequence

<220>
<223>   chimeric anti-CD154 antibody light chain (VL-CL)

<400>   2
Asp Ile Gln Leu Thr Gln Ser Pro Ala Ser Leu Ala Val Ser Leu Gly
1               5                   10                  15

Gln Arg Ala Thr Ile Ser Cys Arg Ala Ser Gln Arg Val Ser Ser Ser
            20                  25                  30

Thr Tyr Ser Tyr Met His Trp Tyr Gln Gln Lys Pro Gly Gln Pro Pro

35                  40                  45

Lys Leu Leu Ile Lys Tyr Ala Ser Asn Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ser Ala Thr Tyr Tyr Cys Gln His Ser Trp
                85                  90                  95

Glu Ile Pro Pro Thr Phe Gly Gly Gly Thr Lys Leu Glu Ile Lys Arg
            100                 105                 110

Ala Asp Ala Ala Pro Thr Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215


<210>    3
<211>    446
<212>    PRT
<213>    Artificial Sequence

<220>
<223>    humanized anti-CD154 antibody heavy chain


<400>    3
Gln Val Gln Leu Val Gln Ser Gly Ala Glu Val Val Lys Pro Gly Ala
1               5                   10                  15

Ser Val Lys Leu Ser Cys Lys Ala Ser Gly Tyr Ile Phe Thr Ser Tyr
            20                  25                  30

Tyr Met Tyr Trp Val Lys Gln Ala Pro Gly Gln Gly Leu Glu Trp Ile
        35                  40                  45

Gly Glu Ile Asn Pro Ser Asn Gly Asp Thr Asn Phe Asn Glu Lys Phe
    50                  55                  60

Lys Ser Lys Ala Thr Leu Thr Val Asp Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Ser Asp Gly Arg Asn Asp Met Asp Ser Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ser His Thr Gln Pro Leu Gly Val Phe
225                 230                 235                 240

Leu Phe Pro Pro Lys Pro Lys Asp Gln Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Leu His Glu Ala Leu His
            420                 425                 430

Asn His Tyr Thr Gln Lys Ser Leu Ser Leu Ser Leu Gly Lys
        435                 440                 445

<210>    4
<211>    218
<212>    PRT
<213>    Artificial Sequence

<220>
<223>    humanized anti-CD154 antibody light chain (VL-CL)

<400>    4
Asp Ile Val Leu Thr Gln Ser Pro Ala Thr Leu Ser Val Ser Pro Gly
  1               5                  10                  15

Glu Arg Ala Thr Ile Ser Cys Arg Ala Ser Gln Arg Val Ser Ser Ser
             20                  25                  30

Thr Tyr Ser Tyr Met His Trp Tyr Gln Gln Lys Pro Gly Gln Pro Pro
         35                  40                  45

Lys Leu Leu Ile Lys Tyr Ala Ser Asn Leu Glu Ser Gly Val Pro Ala
     50                  55                  60

Arg Phe Ser Gly Ser Gly Ser Gly Thr Asp Phe Thr Leu Thr Ile Ser
 65                  70                  75                  80

Ser Val Glu Pro Glu Asp Phe Ala Thr Tyr Tyr Cys Gln His Ser Trp
                 85                  90                  95

Glu Ile Pro Pro Thr Phe Gly Gly Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>    5
<211>    214
<212>    PRT
<213>    Artificial Sequence

<220>
<223>    modified Fc region (NTIG)


<400>    5
Ser His Thr Gln Pro Leu Gly Val Phe Leu Phe Pro Pro Lys Pro Lys
1               5                   10                  15

Asp Gln Leu Met Ile Ser Arg Thr Pro Glu Val Thr Cys Val Val Val
            20                  25                  30

Asp Val Ser Gln Glu Asp Pro Glu Val Gln Phe Asn Trp Tyr Val Asp
        35                  40                  45

Gly Val Glu Val His Asn Ala Lys Thr Lys Pro Arg Glu Glu Gln Phe
    50                  55                  60

Asn Ser Thr Tyr Arg Val Val Ser Val Leu Thr Val Leu His Gln Asp
65                  70                  75                  80

Trp Leu Asn Gly Lys Glu Tyr Lys Cys Lys Val Ser Asn Lys Gly Leu
                85                  90                  95

Pro Ser Ser Ile Glu Lys Thr Ile Ser Lys Ala Lys Gly Gln Pro Arg
            100                 105                 110

Glu Pro Gln Val Tyr Thr Leu Pro Pro Ser Gln Glu Glu Met Thr Lys
        115                 120                 125

Asn Gln Val Ser Leu Thr Cys Leu Val Lys Gly Phe Tyr Pro Ser Asp
    130                 135                 140

Ile Ala Val Glu Trp Glu Ser Asn Gly Gln Pro Glu Asn Asn Tyr Lys
145                 150                 155                 160

Thr Thr Pro Pro Val Leu Asp Ser Asp Gly Ser Phe Phe Leu Tyr Ser
                165                 170                 175

Arg Leu Thr Val Asp Lys Ser Arg Trp Gln Glu Gly Asn Val Phe Ser
            180                 185                 190

Cys Ser Val Leu His Glu Ala Leu His Asn His Tyr Thr Gln Lys Ser
        195                 200                 205

Leu Ser Leu Ser Leu Gly
    210


<210>    6
<211>    215
<212>    PRT
<213>    Artificial Sequence

<220>
<223>    modified Fc region (NTIG)


<400>    6
Ser His Thr Gln Pro Leu Gly Val Phe Leu Phe Pro Pro Lys Pro Lys
1               5                   10                  15

Asp Gln Leu Met Ile Ser Arg Thr Pro Glu Val Thr Cys Val Val Val

            20                  25                  30

Asp Val Ser Gln Glu Asp Pro Glu Val Gln Phe Asn Trp Tyr Val Asp
        35                  40                  45

Gly Val Glu Val His Asn Ala Lys Thr Lys Pro Arg Glu Glu Gln Phe
    50                  55                  60

Asn Ser Thr Tyr Arg Val Val Ser Val Leu Thr Val Leu His Gln Asp
65                  70                  75                  80

Trp Leu Asn Gly Lys Glu Tyr Lys Cys Lys Val Ser Asn Lys Gly Leu
                85                  90                  95

Pro Ser Ser Ile Glu Lys Thr Ile Ser Lys Ala Lys Gly Gln Pro Arg
            100                 105                 110

Glu Pro Gln Val Tyr Thr Leu Pro Pro Ser Gln Glu Glu Met Thr Lys
        115                 120                 125

Asn Gln Val Ser Leu Thr Cys Leu Val Lys Gly Phe Tyr Pro Ser Asp
    130                 135                 140

Ile Ala Val Glu Trp Glu Ser Asn Gly Gln Pro Glu Asn Asn Tyr Lys
145                 150                 155                 160

Thr Thr Pro Pro Val Leu Asp Ser Asp Gly Ser Phe Phe Leu Tyr Ser
                165                 170                 175

Arg Leu Thr Val Asp Lys Ser Arg Trp Gln Glu Gly Asn Val Phe Ser
            180                 185                 190

Cys Ser Val Leu His Glu Ala Leu His Asn His Tyr Thr Gln Lys Ser
        195                 200                 205

Leu Ser Leu Ser Leu Gly Lys
    210                 215


<210> 7
<211> 169
<212> PRT
<213> Artificial Sequence

<220>
<223> monomeric IL-10 variant


<400> 7
Ser Pro Gly Gln Gly Thr Gln Ser Glu Asn Ser Cys Thr His Phe Pro
1               5                   10                  15

Gly Asn Leu Pro Asn Met Leu Arg Asp Leu Arg Asp Ala Phe Ser Arg
            20                  25                  30

Val Lys Thr Phe Phe Gln Met Lys Asp Gln Leu Asp Asn Leu Leu Leu
        35                  40                  45

Lys Glu Ser Leu Leu Glu Asp Phe Lys Gly Tyr Leu Gly Cys Gln Ala
    50                  55                  60

Leu Ser Glu Met Ile Gln Phe Tyr Leu Glu Glu Val Met Pro Gln Ala
65                  70                  75                  80

Glu Asn Gln Asp Pro Asp Ala Lys Ala His Val Asn Ser Leu Gly Glu
                    85                  90                  95

Asn Leu Lys Thr Leu Arg Leu Arg Leu Arg Arg Cys His Arg Phe Leu
            100                 105                 110

Pro Cys Glu Asn Gly Gly Ser Gly Gly Ser Gly Gly Ser Lys Ser Lys
        115                 120                 125

Ala Val Glu Gln Val Lys Asn Ala Phe Asn Lys Leu Gln Glu Lys Gly
    130                 135                 140

Ile Tyr Lys Ala Met Ser Glu Phe Asp Ile Phe Ile Asn Tyr Ile Glu
145                 150                 155                 160

Ala Tyr Met Thr Met Lys Ile Arg Asn
                165


<210>    8
<211>    166
<212>    PRT
<213>    Artificial Sequence

<220>
<223>    prior monomeric IL-10 variant


<400>    8
Ser Pro Gly Gln Gly Thr Gln Ser Glu Asn Ser Cys Thr His Phe Pro
1               5                   10                  15

Gly Asn Leu Pro Asn Met Leu Arg Asp Leu Arg Asp Ala Phe Ser Arg
            20                  25                  30

Val Lys Thr Phe Phe Gln Met Lys Asp Gln Leu Asp Asn Leu Leu Leu
        35                  40                  45

Lys Glu Ser Leu Leu Glu Asp Phe Lys Gly Tyr Leu Gly Cys Gln Ala
    50                  55                  60

Leu Ser Glu Met Ile Gln Phe Tyr Leu Glu Glu Val Met Pro Gln Ala
65                  70                  75                  80

Glu Asn Gln Asp Pro Asp Ala Lys Ala His Val Asn Ser Leu Gly Glu
                85                  90                  95

Asn Leu Lys Thr Leu Arg Leu Arg Leu Arg Arg Cys His Arg Phe Leu
            100                 105                 110

Pro Cys Glu Asn Gly Gly Gly Ser Gly Gly Lys Ser Lys Ala Val Glu
        115                 120                 125

Gln Val Lys Asn Ala Phe Asn Lys Leu Gln Glu Lys Gly Ile Tyr Lys
    130                 135                 140

Ala Met Ser Glu Phe Asp Ile Phe Ile Asn Tyr Ile Glu Ala Tyr Met
145                 150                 155                 160

Thr Met Lys Ile Arg Asn
                165

<210>    9
<211>    216
<212>    PRT
<213>    Artificial Sequence

<220>
<223>    VH-CH1 of chimeric anti-CD154 antibody heavy chain


<400>    9
Glu Val Gln Leu Gln Gln Ser Gly Ser Glu Leu Val Lys Pro Gly Ala
1               5                   10                  15

Ser Val Lys Leu Ser Cys Lys Ala Ser Gly Tyr Ile Phe Thr Ser Tyr
            20                  25                  30

Tyr Met Tyr Trp Val Lys Gln Arg Pro Gly Gln Gly Leu Glu Trp Ile
        35                  40                  45

Gly Glu Ile Asn Pro Ser Asn Gly Asp Thr Asn Phe Asn Glu Lys Phe
    50                  55                  60

Lys Ser Lys Ala Thr Leu Thr Val Asp Lys Ser Ser Ser Thr Ala Tyr
65                  70                  75                  80

Met Gln Leu Ile Ser Leu Thr Ser Glu Asp Ser Ala Val Tyr Tyr Cys
                85                  90                  95

Thr Arg Ser Asp Gly Arg Asn Asp Met Asp Ser Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val
    210                 215


<210>    10
<211>    19
<212>    PRT
<213>    Artificial Sequence

<220>
<223>    signal peptide of IgG heavy chain

<400>    10
Met Gly Trp Ser Cys Ile Ile Leu Phe Leu Val Ala Thr Ala Thr Gly
1               5                   10                  15

Val His Ser

<210>    11
<211>    216
<212>    PRT
<213>    Artificial Sequence

<220>
<223>    VH-CH1 of humanized anti-CD154 heavy chain

<400>    11
Gln Val Gln Leu Val Gln Ser Gly Ala Glu Val Val Lys Pro Gly Ala
1               5                   10                  15

Ser Val Lys Leu Ser Cys Lys Ala Ser Gly Tyr Ile Phe Thr Ser Tyr
            20                  25                  30

Tyr Met Tyr Trp Val Lys Gln Ala Pro Gly Gln Gly Leu Glu Trp Ile
        35                  40                  45

Gly Glu Ile Asn Pro Ser Asn Gly Asp Thr Asn Phe Asn Glu Lys Phe
    50                  55                  60

Lys Ser Lys Ala Thr Leu Thr Val Asp Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Ser Asp Gly Arg Asn Asp Met Asp Ser Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val
    210                 215

<210>    12
<211>    20

<212>    PRT
<213>    Artificial Sequence

<220>
<223>    linker peptide


<400>    12
Ala Ala Gly Ser Gly Gly Gly Gly Gly Ser Gly Gly Gly Gly Ser Gly
1               5                   10                  15

Gly Gly Gly Ser
            20


<210>    13
<211>    20
<212>    PRT
<213>    Artificial Sequence

<220>
<223>    signal peptide of IgG light chain


<400>    13
Met Glu Thr Asp Thr Leu Leu Leu Trp Val Leu Leu Leu Trp Val Pro
1               5                   10                  15

Gly Ser Thr Gly
            20


<210>    14
<211>    119
<212>    PRT
<213>    Artificial Sequence

<220>
<223>    VL of humanized anti-CD154 antibody


<400>    14
Asp Ile Val Leu Thr Gln Ser Pro Ala Thr Leu Ser Val Ser Pro Gly
1               5                   10                  15

Glu Arg Ala Thr Ile Ser Cys Arg Ala Ser Gln Arg Val Ser Ser Ser
            20                  25                  30

Thr Tyr Ser Tyr Met His Trp Tyr Gln Gln Lys Pro Gly Gln Pro Pro
        35                  40                  45

Lys Leu Leu Ile Lys Tyr Ala Ser Asn Leu Glu Ser Gly Val Pro Ala
    50                  55                  60

Arg Phe Ser Gly Ser Gly Ser Gly Thr Asp Phe Thr Leu Thr Ile Ser
65                  70                  75                  80

Ser Val Glu Pro Glu Asp Phe Ala Thr Tyr Tyr Cys Gln His Ser Trp
                85                  90                  95

Glu Ile Pro Pro Thr Phe Gly Gly Gly Thr Lys Leu Glu Ile Lys Arg
            100                 105                 110

Thr Val Ala Ala Pro Ser Val
        115

<210>   15
<211>   118
<212>   PRT
<213>   Artificial Sequence

<220>
<223>   VH of humanized anti-CD154 antibody

<400>   15
Gln Val Gln Leu Val Gln Ser Gly Ala Glu Val Val Lys Pro Gly Ala
1               5                   10                  15

Ser Val Lys Leu Ser Cys Lys Ala Ser Gly Tyr Ile Phe Thr Ser Tyr
            20                  25                  30

Tyr Met Tyr Trp Val Lys Gln Ala Pro Gly Gln Gly Leu Glu Trp Ile
        35                  40                  45

Gly Glu Ile Asn Pro Ser Asn Gly Asp Thr Asn Phe Asn Glu Lys Phe
    50                  55                  60

Lys Ser Lys Ala Thr Leu Thr Val Asp Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Ser Asp Gly Arg Asn Asp Met Asp Ser Trp Gly Gln Gly Thr
            100                 105                 110

Leu Val Thr Val Ser Ser
        115

<210>   16
<211>   15
<212>   PRT
<213>   Artificial Sequence

<220>
<223>   linker peptide

<400>   16
Gly Gly Gly Gly Ser Gly Gly Gly Gly Ser Gly Gly Gly Gly Ser
1               5                   10                  15

<210>   17
<211>   5
<212>   PRT
<213>   Artificial Sequence

<220>
<223>   linker peptide

<400>   17

Gly Gly Ser Gly Gly
1               5

<210>    18
<211>    9
<212>    PRT
<213>    Artificial Sequence

<220>
<223>    linker peptide

<400>    18
Gly Gly Ser Gly Gly Ser Gly Gly Ser
1               5

<210>    19
<211>    6
<212>    PRT
<213>    Artificial Sequence

<220>
<223>    linker peptide

<400>    19
Gly Gly Gly Ser Gly Gly
1               5

<210>    20
<211>    5
<212>    PRT
<213>    Artificial Sequence

<220>
<223>    linker peptide

<400>    20
Gly Gly Gly Gly Ser
1               5

<210>    21
<211>    6
<212>    PRT
<213>    Artificial Sequence

<220>
<223>    linker peptide

<400>    21
Gly Ser Ser Gly Gly Ser
1               5

<210>    22
<211>    18
<212>    PRT

<213>    Artificial Sequence

<220>
<223>    linker peptide


<400>    22
Lys Glu Ser Gly Ser Val Ser Ser Glu Gln Leu Ala Gln Phe Arg Ser
1               5                   10                  15

Leu Asp




<210>    23
<211>    14
<212>    PRT
<213>    Artificial Sequence

<220>
<223>    linker peptide


<400>    23
Glu Gly Lys Ser Ser Gly Ser Gly Ser Glu Ser Lys Ser Thr
1               5                   10


<210>    24
<211>    12
<212>    PRT
<213>    Artificial Sequence

<220>
<223>    linker peptide


<400>    24
Gly Ser Ala Gly Ser Ala Ala Gly Ser Gly Glu Phe
1               5                   10


<210>    25
<211>    5
<212>    PRT
<213>    Artificial Sequence

<220>
<223>    linker peptide


<400>    25
Glu Ala Ala Ala Lys
1               5


<210>    26
<211>    12
<212>    PRT
<213>    Artificial Sequence

<220>
<223>    linker peptide

<400>    26
Cys Arg Arg Arg Arg Arg Arg Glu Ala Glu Ala Cys
1               5                   10


<210>    27
<211>    46
<212>    PRT
<213>    Artificial Sequence

<220>
<223>    linker peptide


<400>    27
Ala Glu Ala Ala Ala Lys Glu Ala Ala Ala Lys Glu Ala Ala Ala Lys
1               5                   10                  15

Glu Ala Ala Ala Lys Ala Leu Glu Ala Glu Ala Ala Ala Lys Glu Ala
            20                  25                  30

Ala Ala Lys Glu Ala Ala Ala Lys Glu Ala Ala Ala Lys Ala
        35                  40                  45



<210>    28
<211>    8
<212>    PRT
<213>    Artificial Sequence

<220>
<223>    linker peptide


<400>    28
Gly Gly Gly Gly Gly Gly Gly Gly
1               5


<210>    29
<211>    6
<212>    PRT
<213>    Artificial Sequence

<220>
<223>    linker peptide


<400>    29
Gly Gly Gly Gly Gly Gly
1               5


<210>    30
<211>    13
<212>    PRT
<213>    Artificial Sequence

<220>
<223>    linker peptide

<400>    30
Ala Glu Ala Ala Ala Lys Glu Ala Ala Ala Ala Lys Ala
1               5                   10


<210>    31
<211>    5
<212>    PRT
<213>    Artificial Sequence

<220>
<223>    linker peptide


<400>    31
Pro Ala Pro Ala Pro
1               5


<210>    32
<211>    17
<212>    PRT
<213>    Artificial Sequence

<220>
<223>    linker peptide


<400>    32
Val Ser Gln Thr Ser Lys Leu Thr Arg Ala Glu Thr Val Phe Pro Asp
1               5                   10                  15

Val


<210>    33
<211>    6
<212>    PRT
<213>    Artificial Sequence

<220>
<223>    linker peptide


<400>    33
Pro Leu Gly Leu Trp Ala
1               5


<210>    34
<211>    10
<212>    PRT
<213>    Artificial Sequence

<220>
<223>    linker peptide


<400>    34
Thr Arg His Arg Gln Pro Arg Gly Trp Glu

1               5                   10


<210>    35
<211>    10
<212>    PRT
<213>    Artificial Sequence

<220>
<223>    linker peptide


<400>    35
Ala Gly Asn Arg Val Arg Arg Ser Val Gly
1               5                   10


<210>    36
<211>    8
<212>    PRT
<213>    Artificial Sequence

<220>
<223>    linker peptide


<400>    36
Arg Arg Arg Arg Arg Arg Arg Arg
1               5


<210>    37
<211>    4
<212>    PRT
<213>    Artificial Sequence

<220>
<223>    linker peptide


<400>    37
Gly Phe Leu Gly
1


<210>    38
<211>    31
<212>    PRT
<213>    Artificial Sequence

<220>
<223>    linker peptide


<400>    38
Gly Ser Ser Gly Gly Ser Gly Ser Ser Gly Gly Ser Gly Gly Gly Asp
1               5                   10                  15

Glu Ala Asp Gly Ser Arg Gly Ser Gln Lys Ala Gly Val Asp Glu
            20                  25                  30

<210>    39
<211>    15
<212>    PRT
<213>    Artificial Sequence

<220>
<223>    linker peptide


<400>    39
Gly Ser Thr Ser Gly Ser Gly Lys Pro Gly Ser Gly Glu Gly Ser
1               5                   10                  15


<210>    40
<211>    15
<212>    PRT
<213>    Artificial Sequence

<220>
<223>    hinge


<400>    40
Glu Pro Lys Ser Cys Asp Lys Thr His Thr Cys Pro Pro Cys Pro
1               5                   10                  15


<210>    41
<211>    15
<212>    PRT
<213>    Artificial Sequence

<220>
<223>    hinge


<400>    41
Glu Pro Lys Ser Ser Asp Lys Thr His Thr Cys Pro Pro Cys Pro
1               5                   10                  15


<210>    42
<211>    8
<212>    PRT
<213>    Artificial Sequence

<220>
<223>    hinge


<400>    42
Thr His Thr Cys Pro Pro Cys Pro
1               5


<210>    43
<211>    33
<212>    PRT
<213>    Artificial Sequence

<220>
<223>    hinge

<400>    43
Gly Gly Gly Gly Ser Gly Gly Gly Gly Ser Gly Gly Gly Gly Ser Glu
1               5                   10                  15

Lys Glu Lys Glu Glu Gln Glu Glu Arg Thr His Thr Cys Pro Pro Cys
            20                  25                  30

Pro

<210>    44
<211>    30
<212>    PRT
<213>    Artificial Sequence

<220>
<223>    hinge

<400>    44
Arg Asn Thr Gly Arg Gly Gly Glu Glu Lys Lys Gly Ser Lys Glu Lys
1               5                   10                  15

Glu Glu Gln Glu Glu Arg Glu Thr Lys Thr Pro Glu Cys Pro
            20                  25                  30

<210>    45
<211>    30
<212>    PRT
<213>    Artificial Sequence

<220>
<223>    hinge

<400>    45
Gly Gly Gly Gly Ser Gly Gly Gly Gly Ser Gly Gly Gly Gly Ser Glu
1               5                   10                  15

Pro Lys Ser Cys Asp Lys Thr His Thr Cys Pro Pro Cys Pro
            20                  25                  30

<210>    46
<211>    26
<212>    PRT
<213>    Artificial Sequence

<220>
<223>    hinge

<400>    46
Gly Ser Gly Gly Gly Ser Gly Thr Leu Val Thr Val Ser Ser Glu Ser
1               5                   10                  15

Lys Tyr Gly Pro Pro Cys Pro Pro Cys Pro
            20                  25

<210>    47
<211>    30
<212>    PRT
<213>    Artificial Sequence

<220>
<223>    hinge


<400>    47
Gly Gly Gly Gly Ser Gly Gly Gly Gly Ser Gly Gly Gly Gly Ser Glu
1               5                   10                  15

Pro Lys Ser Ser Asp Lys Thr His Thr Cys Pro Pro Cys Pro
            20                  25                  30


<210>    48
<211>    55
<212>    PRT
<213>    Artificial Sequence

<220>
<223>    hinge


<400>    48
Gly Gly Gly Gly Ser Gly Gly Gly Gly Ser Gly Gly Gly Gly Ser Ala
1               5                   10                  15

Lys Asn Thr Thr Ala Pro Ala Thr Thr Arg Asn Thr Thr Arg Gly Gly
            20                  25                  30

Glu Glu Lys Lys Lys Glu Lys Glu Lys Glu Glu Gln Glu Glu Arg Thr
        35                  40                  45

His Thr Cys Pro Pro Cys Pro
    50                  55


<210>    49
<211>    39
<212>    PRT
<213>    Artificial Sequence

<220>
<223>    GLP-1/Exendin 4 hybrid


<400>    49
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Gly Pro Ser
            20                  25                  30

Ser Gly Ala Pro Pro Pro Ser
        35

## Description

### TECHNICAL FIELD

The present invention is drawn to a novel fusion protein and use thereof. Particularly, the present invention is drawn to a novel fusion protein that can be used in the treatment of autoimmune diseases and use thereof.

### BACKGROUND ART

Autoimmune reaction is a phenomenon that recognizes cells in the body as foreign substances and destroys them through various immune reactions. Representative autoimmune diseases include rheumatoid arthritis, systemic lupus erythematosus, psoriasis, inflammatory bowel disease, and multiple sclerosis, and these autoimmune diseases are difficult to treat because the cause is not clear. In addition, an autoimmune reaction may occur due to the transplanted organ triggering an immune response. Therefore, development of various immunosuppressants is required to suppress such autoimmune diseases or autoimmune reactions.

Commonly used immunosuppressants include tacrolimus, an inhibitor of calcineurin, mycophenolate mofetil, an inhibitor of gene synthesis, and prednisone, an antiinflammatory steroid. However, since these immunosuppressants should be administered for a long time, they can cause serious side effects (Kalluri and Hardinger, World J. Translant. 2(4): 51-68, 2012; Tanabe et al., Translantation, 93(7): 709-719, 2012; Zaza et al., Toxins 6(3): 869-891, 2014).

Accordingly, various biological drugs are being developed to replace conventional immunosuppressants or increase the duration of drugs, one of them is a costimulation inhibitor. Representative co-stimulatory immune responses include CD28-CD80/CD86, CTLA4-CD80/CD86, CD40-CD40L(CD154), and PD-1/PD-L1, and CTLA4-Fc, anti-CD40 and anti-CD154 antibodies, etc. are currently studied as the above-described biologics in order to inhibit the co-stimulatory immune responses (Kinnear et al., Transplantation 95(4): 527-535, 2013; Riella et al., Am. J. Transplant. 12(10): 2575-2587, 2012; Hardinger and Brennan, World J. Transplant. 3(4): 68-77, 2013). Meanwhile, CD40 is expressed not only in antigen-presenting cells such as B cells, macrophages, dendritic cells, and thymic epithelial cells, but also in normal cells such as epithelial cells and fibroblasts. CD154, also known as CD40L, is primarily expressed in activated T cells and natural killer cells. CD40-CD154 signaling activates B cells and macrophages and indirectly activates T cells, promoting an immune response (Karimi and Pour fathollah, Iran. J. Allergy Asthma Immunol. 11(1): 1-13,2012).

Thus, in order to suppress the immune response by regulating the action of CD154, anti-CD 154 antibodies were developed, and the antibodies showed a good effect in animal experiments including monkeys. However, as a result of clinical trials in humans, anti-CD 154 antibodies exhibited a serious side effect of thrombogenesis, and the development of antibody drugs targeting CD154 is currently suspended (Kalunian et al., Arthritis. Rheum. 46(12): 3251-3258, 2002; Boumpas et al., Arthritis Rheum. 48(3): 719-727, 2003; Pilat et al., Curr. Opin. Organ Translant. 17: 368-375, 2012).

On the other hand, although IL-10 is basically an immune suppressor but it is known to have a dual characteristic that also has the opposite effect of immunostimulatory activity. In this regard, specifically, IL-10 stimulates B cell activation, prolongs the survival of B cells, and may contribute to class switching of B cells. It can also stimulate NK cell proliferation and cytokine production, and may act as a growth factor promoting the proliferation of a specific subset of CD8⁺ T cells (Mosser & Yhang, Immunol. Rev. 226: 205-218, 2009; Cai et al., Eur. J. Immunol. 29: 2658-2665, 1999; Santin et al., J. Virol. 74: 4729-4737, 2000; Rowbottom et al., Immunol. 160: 3188-3193, 1998). Importantly, it has been reported that high doses of IL-10 (20 and 25 µg/kg, respectively) in humans can increase the production of IFNy (Lauw et al., J. Immunol., 165: 2783-2789, 2000; Tilg et al., Gut 50: 191-195, 2002).

The cause of thrombogenesis as described above is known to be due to the binding of anti-CD154 antibodies and FcγRII on platelets surface (Shields et al., J. Biol. Chem. 276(9): 6591-6604, 2001). For this reason, a novel anti-CD154 antibody has been developed by the present inventors to maintain binding to FcγRI and FcyRIII, but block binding to FcyRII, thereby reducing the side effects of thrombogenesis (WO2016/182335A1).

### DISCLOSURE OF THE INVENTION

### TECHNICAL PROBLEM

However, the antibody has a limitation as a medicine in that it cannot completely overcome the side effects of thrombogenesis.

The present invention is to solve various problems including the above-described problems, and the purpose of the present invention is providing a novel anti-CD 154 fusion protein capable of exhibiting a therapeutic effect for autoimmune diseases by effectively blocking the function of CD154 while removing the possibility of thrombogenesis when administered *in vivo.* However, the scope of the present invention is not limited thereto.

### SUMMARY OF THE INVENTION

In an aspect of the present invention, there is provided a fusion protein comprising Fab or scFv specifically binding to human CD154, and an Fc region of immunoglobulin, wherein the Fc region is a modified Fc region mutated so as not to bind to the Fc gamma receptor.

In another aspect of the present invention, there is provided a fusion protein comprising sequentially a) an antigen-binding fragment or an antibody analog of an antibody that specifically binds to CD154; b) a modified Fc region that has been mutated so as not to bind to the Fc gamma receptor; and c) an IL-10 protein.

In another aspect of the present invention, there is provided a polynucleotide encoding the fusion protein.

In another aspect of the present invention, there is provided a vector comprising the polynucleotide.

In another aspect of the present invention, there is provided a pharmaceutical composition for immunosuppression comprising the fusion protein as an active ingredient.

In another aspect of the present invention, there is provided a pharmaceutical composition for treating autoimmune diseases comprising the fusion protein as an active ingredient.

In another aspect of the present invention, there is provided a method for treating a subject suffering from an autoimmune disease comprising administering a therapeutically effective amount of the fusion protein to the subject.

### EFFECT OF THE INVENTION

The fusion protein according to an embodiment of the present invention is a safe material that has a very low possibility of thrombogenesis, which is a disadvantage of the anti-CD 154 antibodies of the prior arts, and can be used as an immunosuppressant and an autoimmune disease treatment.

### BRIEF DESCRIPTION OF DRAWINGS

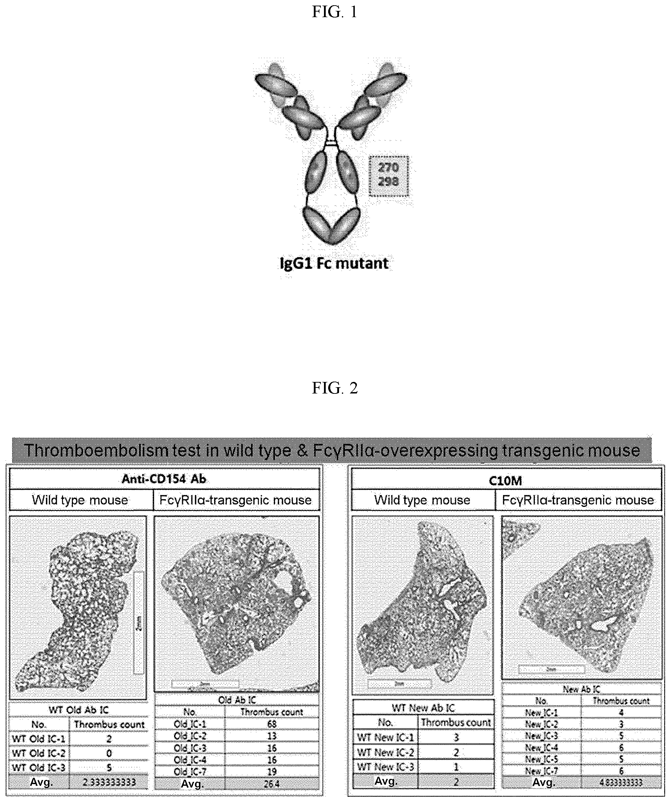

FIG. 1 is a schematic diagram schematically showing the structure of an antibody (C10M) that specifically binds to CD154 described in WO2016/182335A1, which was developed by the present inventors.
FIG. 2 is a series of photographs showing analytic results of staining and counting thrombus generated after administrating an anti-CD 154 antibody to which the wild-type IgG1 Fc region is applied (left) and the anti-CD154 chimeric antibody (C10M, right) to which the variant IgG1 Fc region for inhibiting FcγRIIα binding described in WO2016/182335A1 is applied to wild-type mice and human FcγRIIα-transgenic mice, respectively.

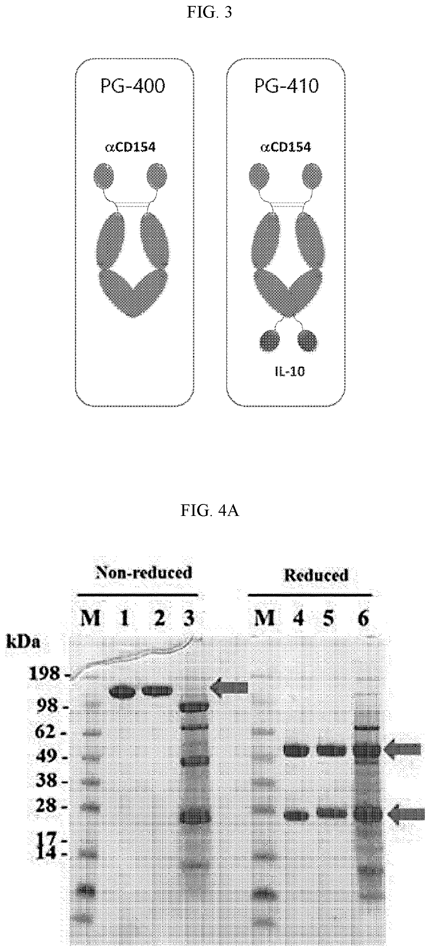

FIG. 3 is a schematic diagram showing the structure of a fusion protein (left: PG-400 and right: PG-410) specifically binding to CD154 according to an embodiment of the present invention.

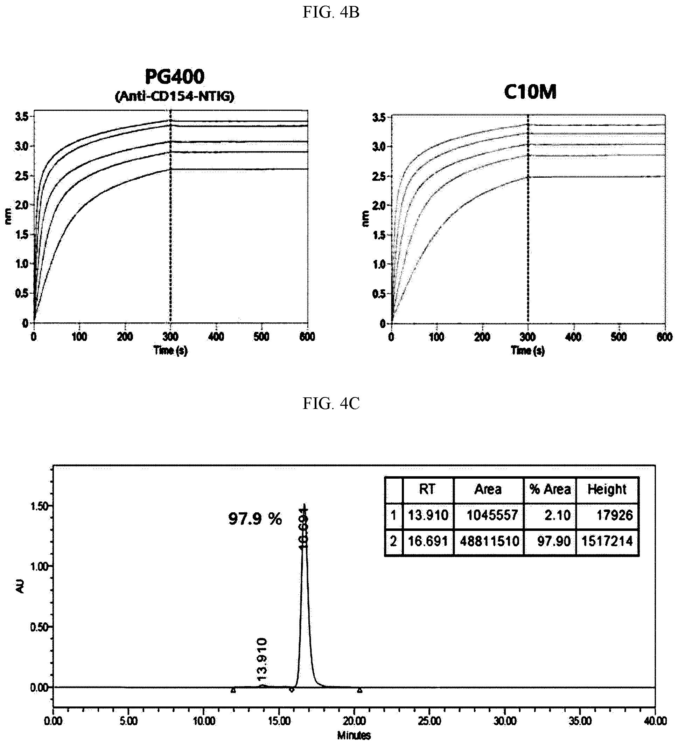

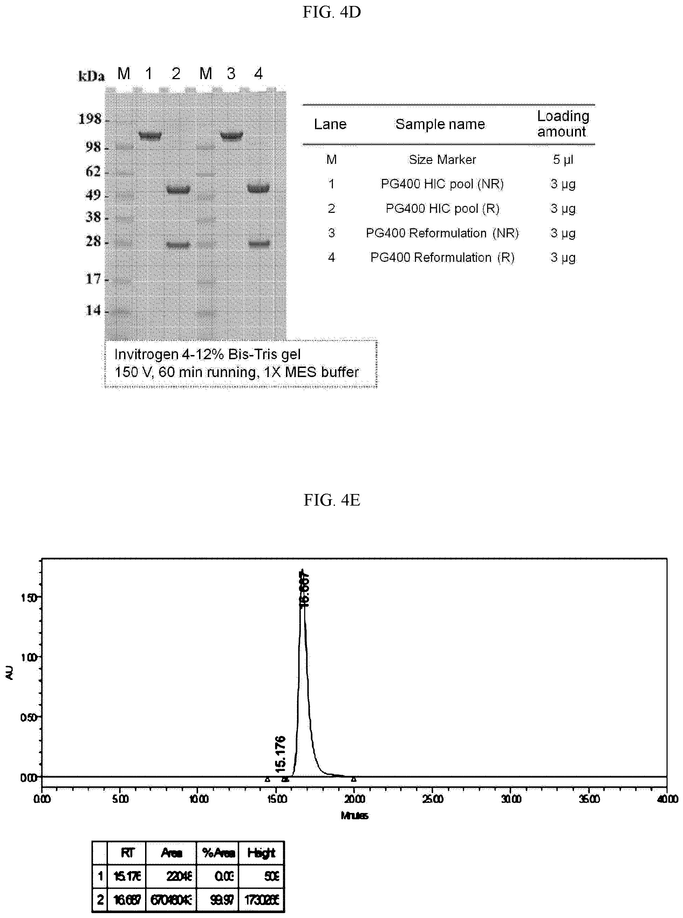

FIG. 4A is a photograph showing a result of analyzing a protein in a purification process by SDS-PAGE after temporarily expressing a chimeric hybrid antibody (PG-400-1) according to an embodiment of the present invention in cells; FIG. 4B is a photograph showing the result of western blot analysis analyzing the chimeric hybrid antibody (PG-400-1) using an anti-IgG4 Fc antibody; FIG. 4C is a chromatogram representing SEC-HPLC analysis performed to measure the purity of the final purified product; FIG. 4D is a photograph showing the results of analyzing the final purified protein by SDS-PAGE after expressing the chimeric hybrid antibody (PG-400-1) from a stable cell line; and FIG. 4E is a chromatogram showing the result of performing SEC-HPLC analysis to confirm the purity of the final protein expressed from stable cell line.

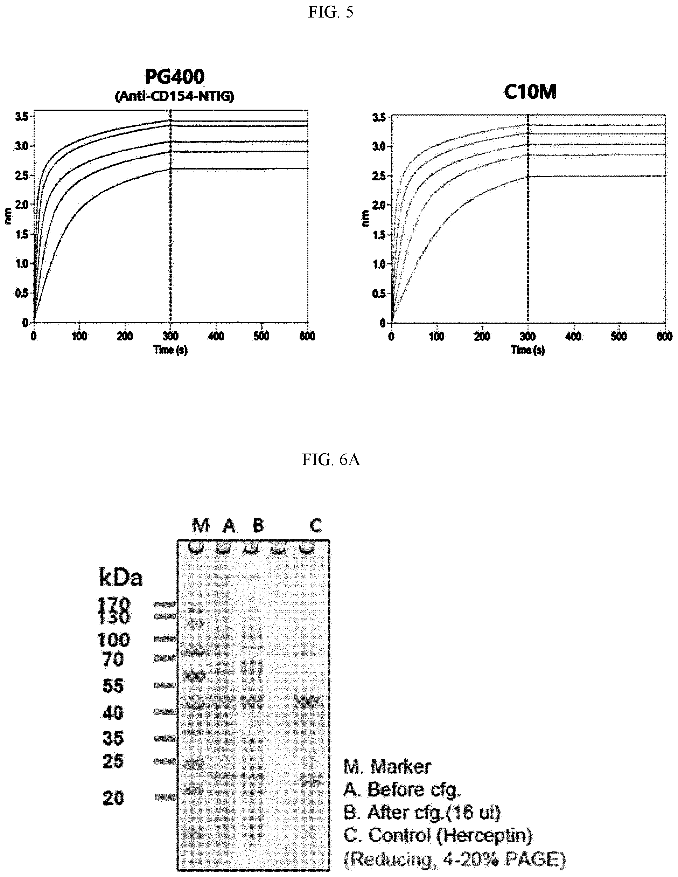

FIG. 5 is a series of graphs representing results of BLI analyses analyzing binding affinity of CD40L and an anti-CD154 chimeric antibody (C10M, right) to which a variant IgG1 Fc region for inhibiting FcγRIIα binding described in WO2016/182335A1 is applied, and a chimeric hybrid antibody prepared in Example 2 (PG-400-1, left), respectively.

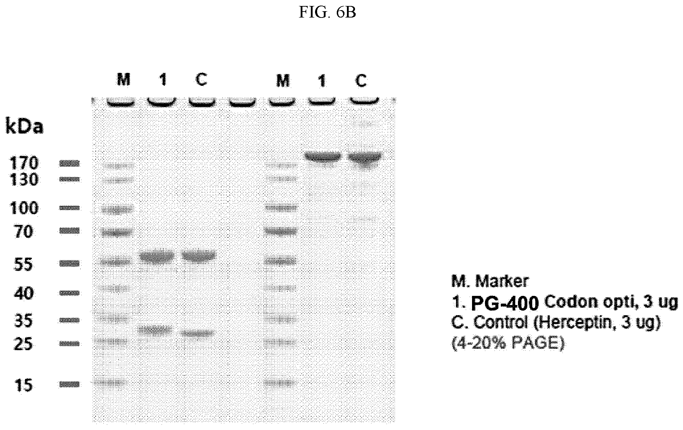

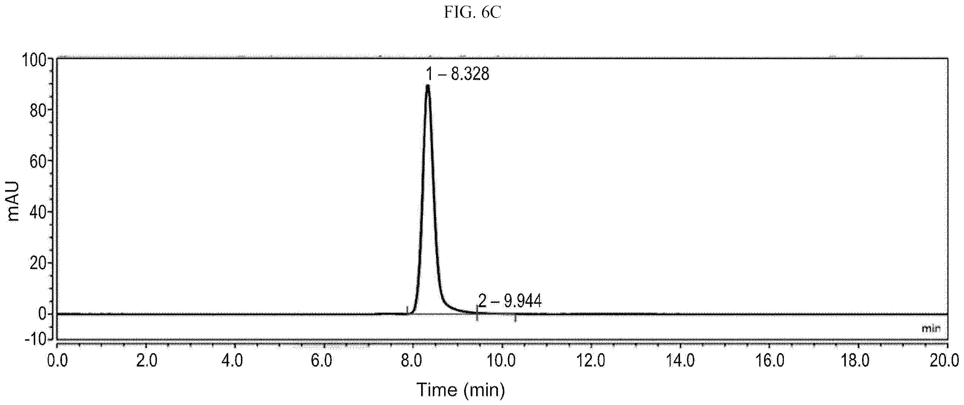

FIG. 6A is a photograph showing the result of reduced PAGE analysis performed on a cell culture solution after expressing a recombinant humanized anti-CD 154 antibody (PG-400-2) according to an embodiment of the present invention in HEK293 cells; and FIG. 6B is a photograph showing the PAGE analyses of protein under reduced (left) and nonreduced (right) conditions after purifying cell culture medium through Protein A affinity chromatography and gel filtration; and FIG. 6C is a chromatogram representing HPLC analysis of purified PG-400-2.

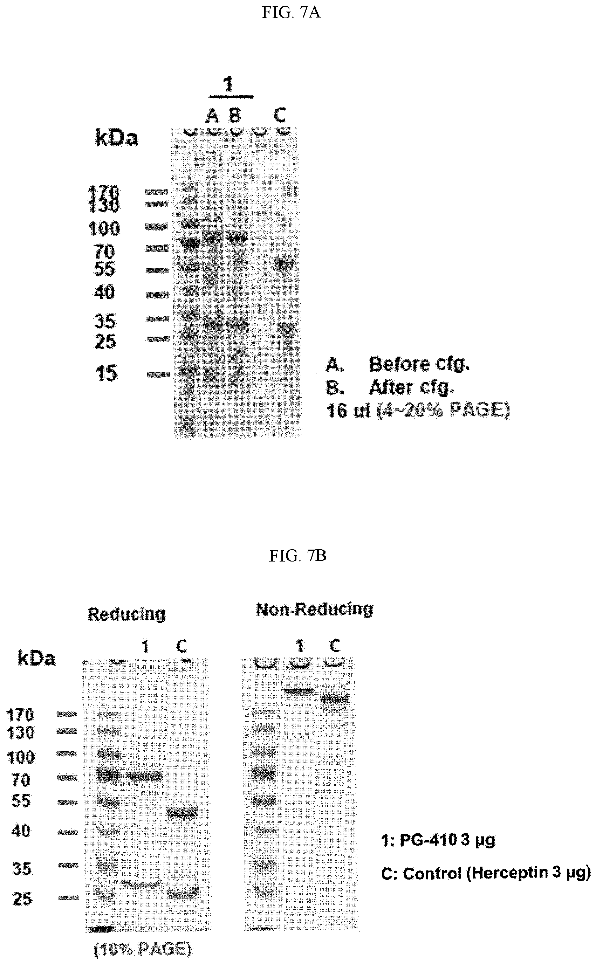

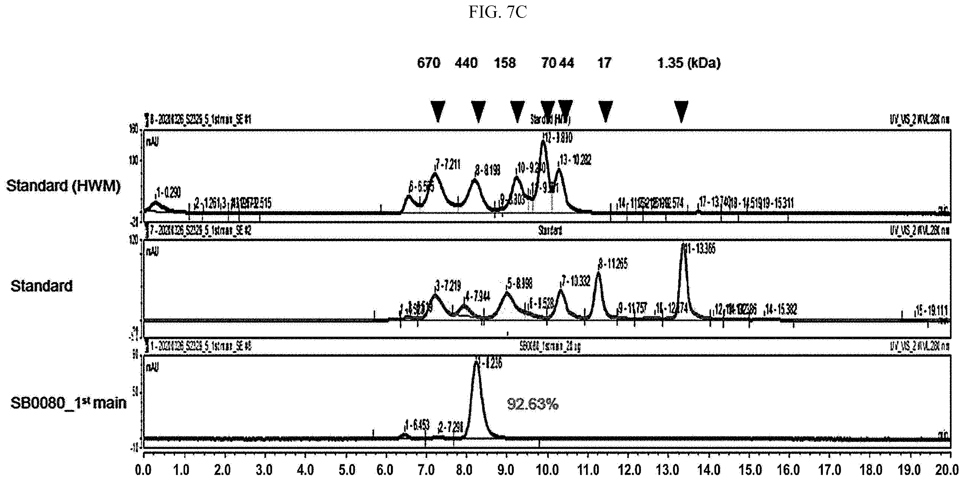

FIG. 7A is a photograph showing the result of a reduced PAGE analysis performed on a cell culture solution after expressing the recombinant humanized anti-CD 154 antibody (PG-410) according to an embodiment of the present invention in HEK293 cells; FIG. 7B is a photograph representing the results of PAGE analysis of protein under reduced (left) and non-reducing (right) conditions after purifying cell culture medium through Protein A affinity chromatography and gel filtration; and FIG. 7C is a chromatogram representing HPLC analysis of purified PG-410.

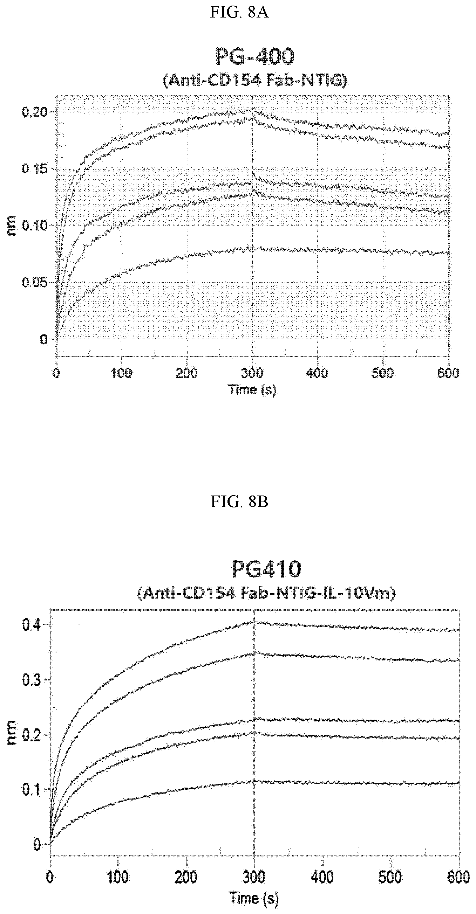

FIG. 8A is a sensogram showing the result of analyzing the binding degree of the recombinant humanized anti-CD 154 antibody (PG-400-2) according to an embodiment of the present invention to CD154 by BLI analysis; FIG. 8B is a sensogram showing the result of analyzing the binding degree of the recombinant humanized anti-CD 154 antibody (PG-410) according to an embodiment of the present invention to CD154 by BLI analysis.

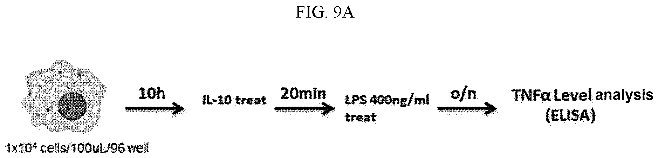

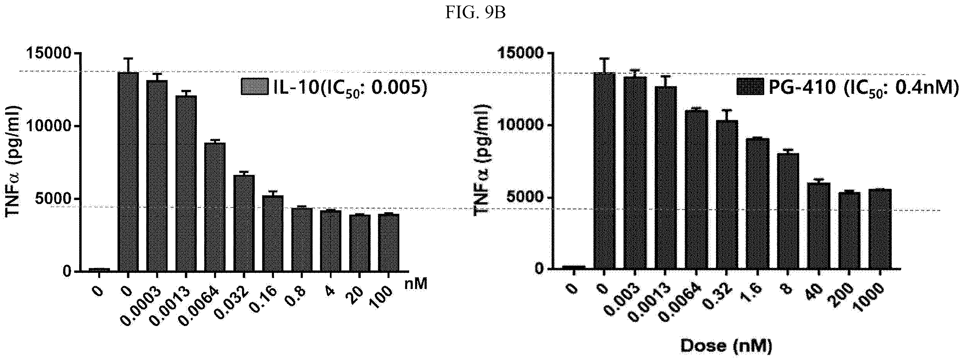

FIG. 9A is a schematic diagram showing an experimental schedule for investigating the immunosuppressive activity by IL-10 of a recombinant humanized anti-CD154 antibody (PG-410) according to an embodiment of the present invention; and FIG. 9B is a graph showing the results of measuring the expression of TNFα when a recombinant IL-10 (left) and the recombinant humanized anti-CD 154 antibody (PG-410) according to an embodiment of the present invention were treated with the macrophage cell line Raw264, respectively.

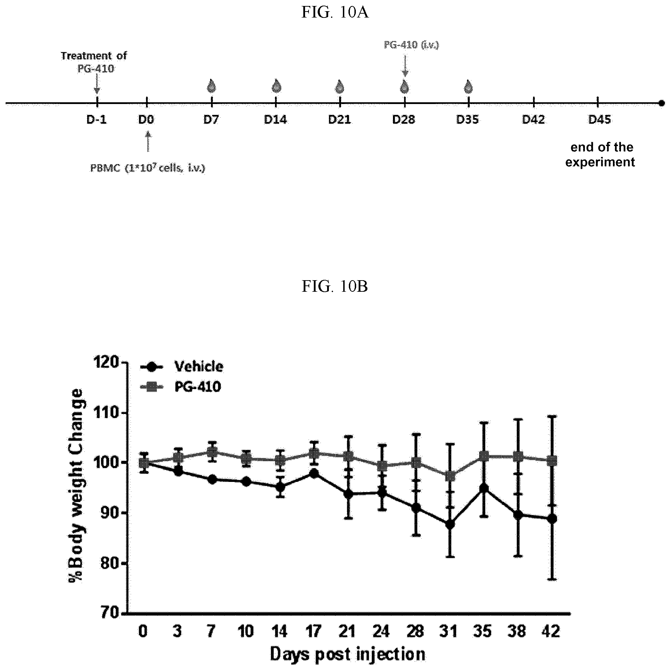

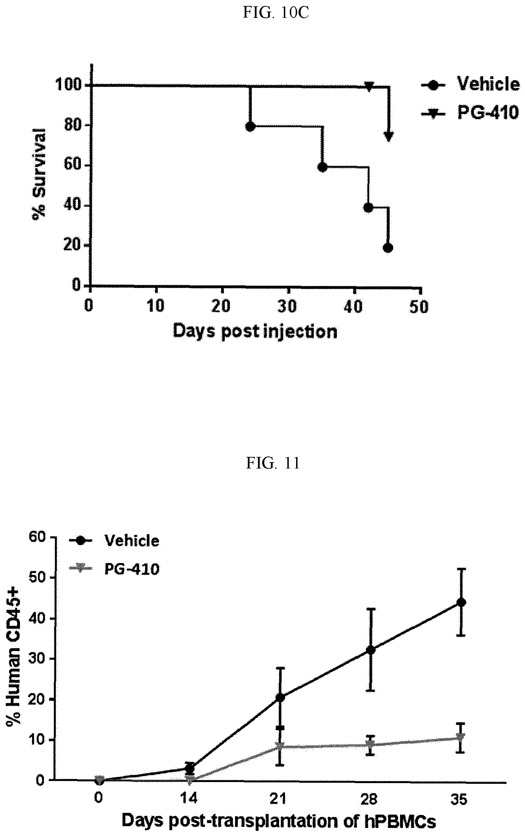

FIG. 10A is a schematic diagram showing an animal experiment schedule for confirming the effect of GVHD treatment by administration of a recombinant humanized anti-CD 154 antibody (PG-410) according to an embodiment of the present invention; FIG. 10B is a graph showing the change in body weight over time in the control group and the PG-410 administration group; and FIG. 10C is a graph showing the survival rate over time in the control group administered with PBMC and the experimental group administrated with PG-410.
FIG. 11 is a graph showing the results of measuring the ratio of human CD45⁺ cell population over time in the control group (vehicle) and the recombinant humanized anti-CD 154 antibody (PG-410) administration group according to an embodiment of the present invention.

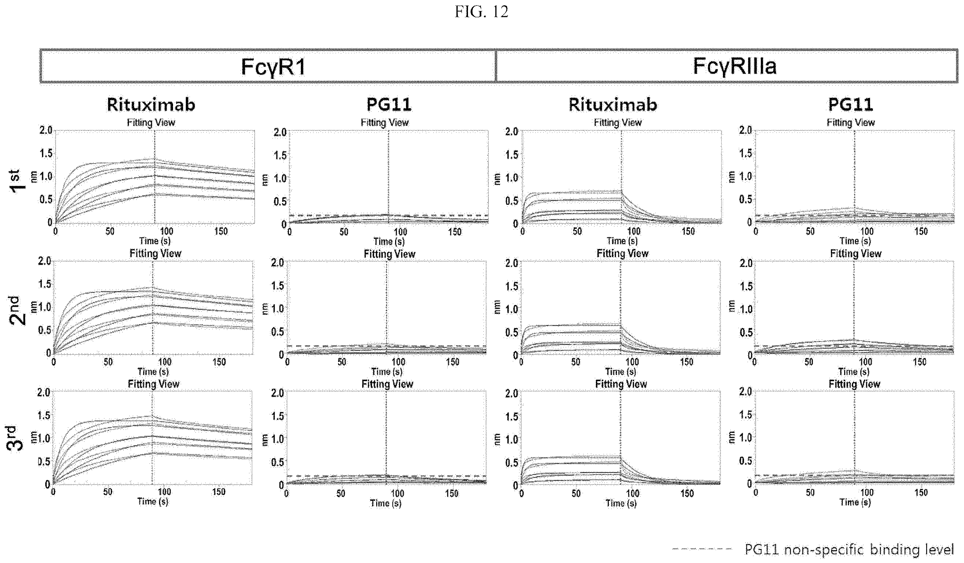

FIG. 12 is a series of sensograms showing the results of BLI analysis comparing the binding affinity of the modified Fc region protein according to an embodiment of the present invention and rituximab (control) to Fc gamma receptors I and IIIa.

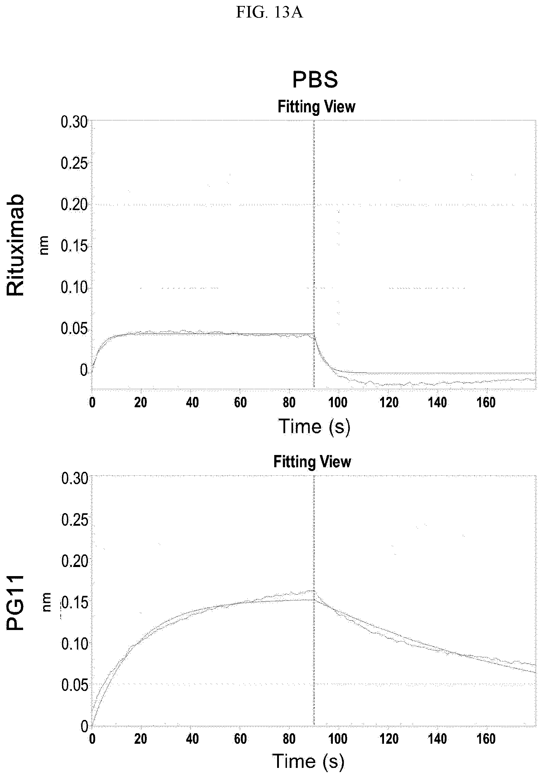

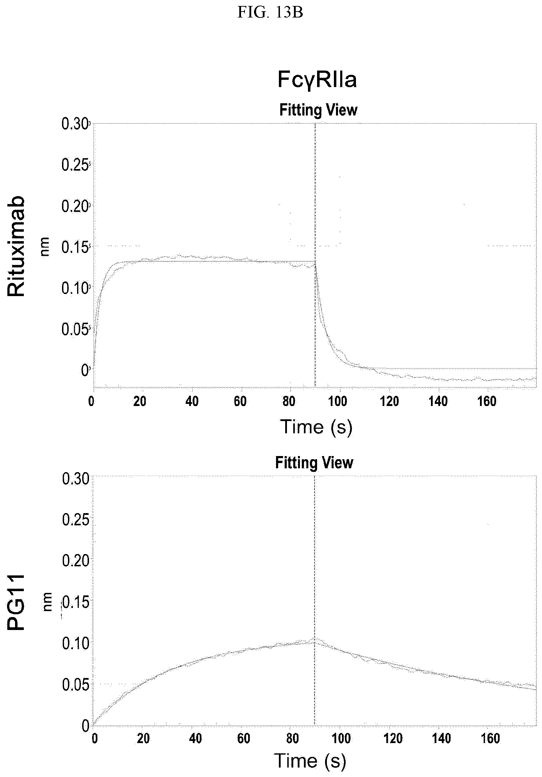

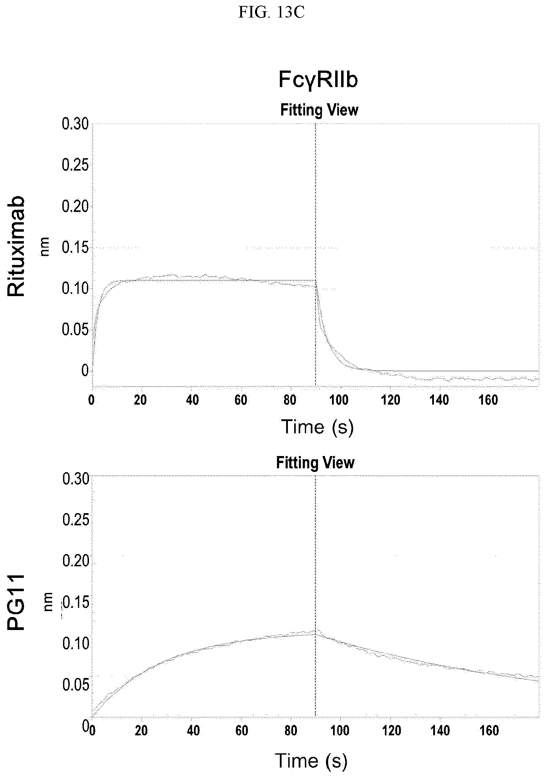

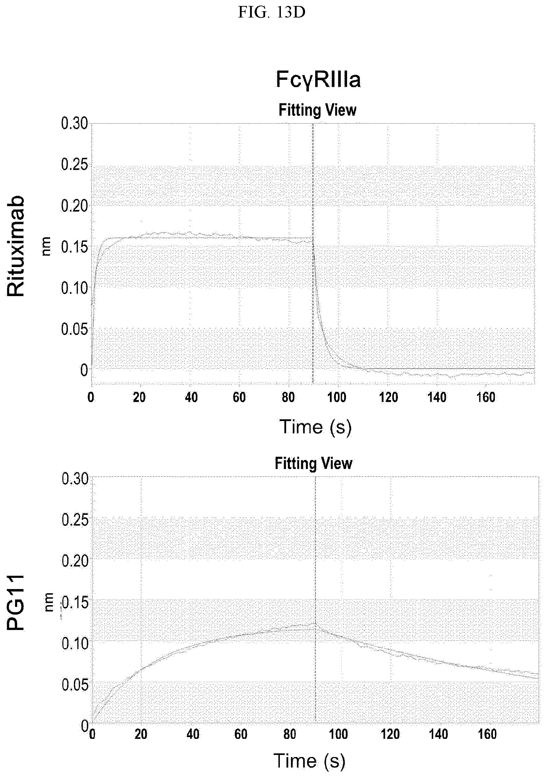

FIG. 13 shows a result of comparative analysis of binding affinity for various Fc gamma receptors of a modified Fc region protein according to an embodiment of the present invention and rituximab (control); FIG. 13A is a sensogram showing the result of BLI analysis after treating with PBS in order to confirm a baseline; FIG. 13B is a sensogram showing the result of BLI analysis comparing the binding affinity of the modified Fc region protein according to an embodiment of the present invention and rituximab (control) to Fc gamma receptor IIa; FIG. 13C is a sensogram showing the result of BLI analysis comparing the binding affinity of the modified Fc region protein according to an embodiment of the present invention and rituximab (control) with Fc gamma receptor IIb; and FIG. 13D is a sensogram showing the result of BLI analysis comparing the binding affinity of the modified Fc region protein according to an embodiment of the present invention and rituximab (control) with the Fc gamma receptor IIIb.

### BEST MODES OF THE INVENTION

In an aspect of the present invention, there is provided a fusion protein comprising Fab or scFv specifically binding to human CD154, and an Fc region of immunoglobulin, wherein the Fc region is a modified Fc region mutated so as not to bind to the Fc gamma receptor.

In the fusion protein, the Fab specifically binding to CD154 may consist of a light chain (V_{L}-C_{L}) consisting of an amino acid represented by SEQ ID NO: 2 and a heavy chain V_{H}-CH1 fragment consisting of an amino acid represented by SEQ ID NO: 9; or a light chain (V_{L}-C_{L}) consisting of the amino acid sequence represented by SEQ ID NO: 4 and a heavy chain V_{H}-CH1 fragment consisting of the amino acid sequence represented by SEQ ID NO: 11.

In the fusion protein, the scFv may be prepared by linking a light chain variable domain (V_{L}) consisting of an amino acid sequence represented by SEQ ID NO: 14 and a heavy chain variable region (V_{H}) consisting of an amino acid sequence represented by SEQ ID NO: 15 with a linker peptide. As the linker peptide, any one of those described below may be used.

In the fusion protein, the Fc gamma receptor may be FcγRI, FcyRIIA, FcγRIIB1, FcγRIIB2, FcyRIIIA, and/or FcγRIIIB.

In the fusion protein, the Fab or scFv specifically binding to CD154, and the Fc region may be linked by a hinge or linker peptide. The hinge may be derived from an IgG1, IgG2, IgG3, IgG4, IgM, IgD, or a hybrid hinge of at least two or more of them are mixed. The hinge may include the amino acid sequence set forth in any one of SEQ ID NOs: 40 to 42. Any one of the linker peptides may be used as long as the linker peptide does not inhibit the function of the fusion partner of the fusion protein, and preferably those defined below may be used.

In the fusion protein, the Fc region may be mutated so as not to substantially bind to the Fc gamma receptor, and any of the conventionally known variants of Fc region which do not bind to the Fc gamma receptor may be used, and it may be derived from IgA, IgG, IgM, IgD or IgE or may be a hybrid Fc in which Fc regions of the particular immunoglobulins or domains thereof, for example, all or parts of CH2 and CH3 are mixed. The IgG may be IgG1, IgG2, IgG3, or IgG4. More specifically, the Fc region may be a variant Fc whose functional parts (effectors) responsible for antibody-dependent cell-mediated cytotoxicity (ADCC) or complement-dependent cytotoxicity (CDC) are mutated in order to lower affinity for Fc gamma receptor (FcyRc) and a complement (C1a) and/or a variant Fc engineered to improve selective affinity to neonatal Fc receptor (FcRn) having elevated blood half-life thereby. Among these, the hybrid Fc may be those described in Korean Patent Nos. 897938, 1380732, 1380729, etc., and the variant Fc may be a modified immunoglobulin Fc protein (NTIG) described in International Patent Application PCT/KR2020/006346. Preferably, the Fc region may be a modified Fc region having the amino acid sequence of SEQ ID NO: 5 or 6, or modified Fc region in which 18^{th} and 196^{th} amino acids are substituted with threonine (T) and methionine (M), respectively in the amino acid sequence of SEQ ID NOs: 5 or 6.

The fusion protein may be a form in which an IL-10 protein is added to the C-terminus of the Fc region.

The IL-10 protein may be a mature form protein comprising an amino acid sequence 19^{th} to 178^{th} in which a signal sequence has been removed from the amino acid sequence described in UniProtKB P22301, and it may be a IL-10 variant protein in which isoleucine, the 87^{th} amino acid of the mature protein, is substituted with alanine and it may be a monomeric IL-10 variant protein in which a linker (spacer) peptide with a length of 6 to 12 a.a. is inserted between asparagine, the 116^{th} amino acid and lysine, the 117^{th} amino acid.

In another aspect of the present invention, there is provided a fusion protein comprising sequentially a) an antigen-binding fragment or an antibody analog of an antibody that specifically binds to CD154; b) a modified Fc region that has been mutated so as not to bind to the Fc gamma receptor; and c) an IL-10 protein. In the fusion protein, the antigen-binding fragment of the antibody may be Fab, F(ab')₂ , Fab', scFv, diabody, triabody, sdAb (single domain antibody), V_{NAR} or V_{H}H, and the antibody analog may be affibody, affilin, affimer, affitin, alphabody, anticalin, avimer, DARPin, Fynomer, Kunitz domain peptide, monobody, repebody, VLR, or nanoCLAMP

In the fusion protein, the Fc gamma receptor may be FcγRI, FcγRIIA, FcyRIIB 1, FcγRIIB2, FcyRIIIA, and/or FcγRIIIB.

In the fusion protein, between a) and b) and between b) and c) may be connected by a hinge or a linker peptide. The hinge may be derived from an IgG1, IgG2, IgG3, IgG4, IgM, IgD, or a hybrid hinge of at least two or more of them are mixed. The hinge may include the amino acid sequence set forth in any one of SEQ ID NOs: 40 to 42. Any one of the linker peptides may be used as long as the linker peptide does not inhibit the function of the fusion partner of the fusion protein, and preferably those defined below may be used.

In the fusion protein, the Fc region may be mutated so as not to substantially bind to the Fc gamma receptor, and any of the conventionally known variants of Fc region which do not bind to the Fc gamma receptor may be used. Preferably, the Fc region may be a modified Fc region having the amino acid sequence of SEQ ID NOs: 5 or 6, or modified Fc region in which 18^{th} and 196^{th} amino acids are substituted with threonine (T) and methionine (M), respectively in the amino acid sequence of SEQ ID Nos: 5 or 6.

In the fusion protein, the IL-10 protein may be a mature form protein comprising an amino acid sequence 19^{th} to -178^{th} in which a signal sequence has been removed from the amino acid sequence described in UniProtKB P22301, and it may be a IL-10 variant protein in which isoleucine, the 87^{th} amino acid of the mature protein, is substituted with alanine and it may be a monomeric IL-10 variant protein in which a linker (spacer) peptide with a length of 6 to 12 a.a. is inserted between asparagine, the 116^{th} amino acid and lysine, the 117^{th} amino acid. The IL-10 variant protein may consist of an amino acid sequence represented by SEQ ID NO: 7 or 8.

As used herein, the term "fusion protein" refers to a recombinant protein in which two or more proteins or domains responsible for specific functions in a protein are linked such that each protein or domain is responsible for an original function thereof. A linker peptide having a flexible structure can be generally inserted between the two or more proteins or domains. The linker peptide may be AAGSGGGGGSGGGGSGGGGS (SEQ ID NO: 12), GGGGSGGGGSGGGGS (SEQ ID NO: 16), GGSGG (SEQ ID NO: 17), GGSGGSGGS (SEQ ID NO: 18), GGGSGG (SEQ ID NO: 19), (G₄S)ₙ (unit: SEQ ID NO: 20, n is an integer of 1 to 10), (GGS)ₙ (n is an integer of 1 to 10), (GS)ₙ (n is an integer of 1 to 10), (GSSGGS)ₙ (unit: SEQ ID NO: 21, n is an integer of 1 to 10), KESGSVSSEQLAQFRSLD (SEQ ID NO: 22), EGKSSGSGSESKST (SEQ ID NO: 23), GSAGSAAGSGEF (SEQ ID NO: 24), (EAAAK)ₙ (unit: SEQ ID NO: 25, n is an integer of 1 to 10), CRRRRRREAEAC (SEQ ID NO: 26), A(EAAAK)₄ALEA(EAAAK)₄A (SEQ ID NO: 27), GGGGGGGG (SEQ ID NO: 28), GGGGGG (SEQ ID NO: 29), AEAAAKEAAAAKA (SEQ ID NO: 30), PAPAP (SEQ ID NO: 31), (Ala-Pro)n (n is an integer from 1 to 10), VSQTSKLTRAETVFPDV (SEQ ID NO: 32), PLGLWA (SEQ ID NO: 33), TRHRQPRGWE (SEQ ID NO: 34), AGNRVRRSVG (SEQ ID NO: 35), RRRRRRRR (SEQ ID NO: 36), GFLG (SEQ ID NO: 37), GSSGGSGSSGGSGGGDEADGSRGSQKAGVDE (SEQ ID NO: 38), GSTSGSGKPGSGEGS (SEQ ID NO: 39), EPKSCDKTHTCPPC (SEQ ID NO: 40), EPKSSDKTHGGTCPPC (SEQ ID NO: 41) and THTCPPKPGS (SEQ ID NO: 42), RNTGRGGEEKKGSKEKEEQEERETKTPECP (SEQ ID NO: 44), GGGGSGGGGSGGGGSEPKSCDKTHTCPPCP (SEQ ID NO: 45), GSGGGSGTLVTVSSESKYGPPCPPCP (SEQ ID NO: 46), GGGGSGGGGSGGGGSEPKSSDKTHTCPPCP (SEQ ID NO: 47), and GGGGSGGGGSGGGGSAKNTTAPATTRNTTRGGEEKKKEKEKEEQEERTHTCPPCP (SEQ ID NO: 48), and the like. In particular, SEQ ID NOs: 40 to 48 are hinge peptides derived from an antibody or a hybrid hinge peptide in which a hinge and other linker peptides are mixed. It is advantageous in that the basic structure of the antibody is utilized as it is when a Fab specifically binding to CD154 is linked to the Fc region.

As used herein, the term "antibody" refers to an immunoglobulin molecule which is a hetero-tetrameric protein produced by binding two identical heavy chains and two identical light chains, and performs antigen-specific binding through an antigen-binding site composed of a variable region (V_{L}) of the light chain and a variable region (V_{H}) of the heavy chain, thereby causing an antigen-specific humoral immune response.

As used herein, the term "antigen-binding fragment of an antibody" refers to a fragment which has antigen-binding ability derived from an antibody and includes both a fragment produced by cleaving an antibody with a protein cleaving enzyme as well as a single-chain fragment produced in a recombinant manner, and examples thereof include Fab, F(ab')₂, scFv, diabody, triabody, sdAb, or V_{H}H.

As used herein, the term "Fab" refers to an antigen-binding antibody fragment (fragment antigen-binding) which is produced by cleaving an antibody molecule with a proteolytic enzyme, papain, is a heterodimer of two peptides of V_{H}-CH1 and V_{L}-C_{L}, and the other fragment produced by the papain is referred to as Fc (fragment crystallizable).

As used herein, the term "F(ab')₂" refers to a fragment that includes an antigen-binding site among fragments produced by cleaving an antibody with pepsin, which is a proteinase, and is in a form of a tetramer in which the two Fab's are linked by a disulfide bond. The other fragment produced by the pepsin is referred to as pFc'.

As used herein, the term "Fab" refers to a molecule having a similar structure to that of Fab produced by separating the abovementioned F(ab')₂ under weak reducing conditions.

As used herein, the term "scFv" is an abbreviation for a "single chain variable fragment", and refers to a fragment which is not a fragment of an actual antibody, but is a kind of fusion protein prepared by linking the heavy-chain variable region (V_{H}) to the light-chain variable region (V_{L}) of the antibody through a linker peptide having a size of about 25 a.a., and is known to have antigen-binding ability even though the fragment is not a unique antibody fragment (Glockshuber et al., Biochem. 29(6): 1362-1367, 1990).

As used herein, the terms "diabody" and "triabody" refer to antibody fragments in a form of two and three scFv's linked by a linker, respectively.

As used herein, the term "single domain antibody (sdAb)" refers to an antibody fragment which is also referred to as a nanobody and consists of a single variable region fragment of an antibody. The sdAb derived from the heavy chain is mainly used, but a single variable region fragment derived from the light chain is also reported to specifically bind to an antigen. V_{NAR} composed of variable region fragments of a shark antibody and V_{H}H composed of variable region fragments of a camelid antibody, which consist only of dimers of single chains unlike conventional antibodies composed of a heavy chain and a light chain, are also included in sdAb.

As used herein, the term "antibody mimetic" or alternatively "antibody analog" is a concept including a protein having similar functions to those of antibodies prepared from non-antibody-derived protein scaffolds such as a monobody and a variable lymphocyte receptor (VLR), that is, having antigen-binding ability, unlike a normal full-length antibody in which two heavy chains and two light chains form a quaternary structure of a hetero-tetramer to exhibit functions. Examples of such an antibody mimetic include Affibody derived from a Z domain of protein A (Nygren, P. A., FEBS J. 275(11): 2668 to 2676, 2008), Affilin derived from Gamma-B crystallin or Ubiquitin (Ebersbach et al., J. Mol. Biol. 372(1): 172-185, 2007), Affimer derived from Cystatin (Johnson et al., Anal. Chem. 84(15): 6553-6560, 2012), Affitin derived from Sac7d (Krehenbrink et al., J. Mol. Biol. 383 (5): 1058-1068, 2008), Alphabody derived from a triple helix coiled coil protein (Desmet et al., Nat. Commun. 5: 5237, 2014), Anticalin derived from lipocalin (Skerra et al., FEBS J. 275(11): 2677-2683, 2008), Avimer derived from domains of various membrane receptors (Silverman et al., Nat. Biotechnol. 23(12): 1556-1561, 2005), DARPin derived from Ankyrin repeat motif (Stumpp et al., DrugDiscov. Today. 13(15-16): 695-701, 2008), Fynomer derived from a SH3 domain of a Fyn protein (Grabulovski et al., J. Biol. Chem. 282(5): 3196-3204, 2007), Kunitz domain peptides derived from Kunitz domains of various protein inhibitors (Nixon and Wood, Curr. Opin. Drug Discov. Dev. 9(2): 261-268, 2006), a monobody derived from the 10^{th} type 3 domain of fibronectin (Koide and Koide, Methods Mol. Biol. 352: 95-109, 2007), nanoCLAMP derived from carbohydrate-binding module 32-2 (Suderman et al., Protein Exp. Purif. 134: 114-124, 2017), a variable lymphocyte receptor (VLR) derived from a hagfish *(*Boehm et al., Ann. Rev. Immunol. 30: 203-220, 2012), and a repebody engineered to enhance antigen affinity based on the VLR (Lee et al., Proc. Natl. Acad. Sci. USA, 109: 3299-3304, 2012).

In another aspect of the present invention, there is provided a polynucleotide encoding the fusion protein.

In another aspect of the present invention, there is provided a recombinant vector comprising the polynucleotide.

In the recombinant vector, the polynucleotide may be contained in a form of a gene construct operably linked to a regulatory sequence.

As used herein, the term "operably linked to" means that a target nucleic acid sequence (for example, *in vitro* transcription/translation system or in a host cell) is linked to the regulatory sequence in such a way that the target nucleic acid sequence can be expressed.

As used herein, the term "regulatory sequence" is meant to include a promoter, an enhancer, and other regulatory elements (for example, polyadenylation signal). Examples of the regulatory sequence include a sequence which directs such that a target nucleic acid is constantly expressed in many host cells, a sequence (for example, a tissue-specific regulatory sequence) which directs such that a target nucleic acid is expressed only in a specific tissue cell, and a sequence (for example, an inducible regulatory sequence) which directs such that expression is induced by a specific signal. Those skilled in the art could understand that the design of an expression vector may vary depending on factors such as the selection of a host cell to be transformed and the desired level of protein expression. The expression vector of the present invention can be introduced into a host cell to express the fusion protein. Regulatory sequences which enable expression in the eukaryotic cell and the prokaryotic cell are well known to those skilled in the art. As described above, these regulatory sequences generally include regulatory sequences responsible for transcription initiation, and optionally, a poly-A signal responsible for transcription termination and stabilization of a transcript. Additional regulatory sequences may include a translation enhancing factor and/or a naturally-combined or heterologous promoter region, in addition to the transcription regulatory factor. For example, possible regulatory sequences which enable expression in a mammalian host cell include a CMV-HSV thymidine kinase promoter, SV40, an RSV (Rous sarcoma virus)-promoter, a human kidney urea 1α-promoter, a glucocorticoid-inducing MMTV (Moloney mouse tumor virus)-promoter, a metallothionein- or tetracycline-inducible promoter, or an amplifying agent such as a CMV amplifying agent and an SV40 amplifying agent. It is considered that for expression in a nerve cell, a neurofilament-promoter, a PGDF-promoter, an NSE-promoter, a PrP-promoter, or a thy-1-promoter can be used. The abovementioned promoters are known in the art, and are described in the literature (Charron, J. Biol. Chem. 270: 25739 to 25745, 1995). For the expression in the prokaryotic cell, a number of promoters, including a lac-promoter, a tac-promoter, or a trp promoter, have been disclosed. In addition to the factors capable of initiating transcription, the regulatory sequences may include a transcription termination signal, such as an SV40-poly-A site and a TK-poly-A site, on the downstream of the polynucleotide according to one exemplary embodiment of the present invention. In the present specification, suitable expression vectors are known in the art, and examples thereof include Okayama-Berg cDNA expression vector pcDV1 (Parmacia), pRc/CMV, pcDNA1, pcDNA3 (Invitrogen), pSPORT1 (GIBCO BRL), pGX-27 (Korean Patent No. 1442254), pX (Pagano et al., Science 255: 1144-1147, 1992), a yeast two-hybrid vector such as pEG202 and dpJG4-5 (Gyuris et al., Cell 75: 791-803, 1995), and a prokaryotic expression vector such as lambda gt11 and pGEX (Amersham Pharmacia). The vector may further include a polynucleotide encoding a secretion signal, in addition to the nucleic acid molecules of the present invention. The secretion signals are well known to those skilled in the art. Moreover, depending on the used expression system, a leader sequence which can lead the fusion protein according to one exemplary embodiment of the present invention to a cellular compartment is combined with a coding sequence of the polynucleotide according to one exemplary embodiment of the present invention, and is preferably a leader sequence capable of directly secreting a decoded protein or the protein thereof into a pericytoplasmic or extracellular medium.

In addition, the vector of the present invention can be prepared, for example, by a standard recombinant DNA technique, and examples of the standard recombinant DNA technique include ligation of a smooth terminus and an adhesion terminus, a restriction enzyme treatment to provide a proper terminus, removal of a phosphate group by an alkaline phosphatase treatment to prevent inappropriate binding, and enzymatic linkage by T4 DNA ligase. The vector of the present invention can be prepared by recombining DNA encoding a signal peptide obtained by chemical synthesis or a genetic recombination technique, the immunoglobulin Fc domain variant protein according to one exemplary embodiment of the present invention, or DNA encoding a fusion protein containing the same with a vector containing an appropriate regulatory sequence. The vector containing a regulatory sequence can be commercially purchased or prepared, and in one exemplary embodiment of the present invention, a pBispecific backbone vector (Genexine, Inc., Korea), a pAD15 vector, pGP30 (Genexine, Inc. Korea), or a pN293F vector (Y-Biologics, Inc., Korea) was used as a backbone vector.

The expression vector may further include a polynucleotide encoding a secretion signal sequence, and the secretion signal sequence induces the extracellular secretion of the recombinant protein expressed in the cell, and may be a tissue plasminogen activator (tPA) signal sequence, a herpes simplex virus glycoprotein Ds (HSV gDs) signal sequence, or a growth hormone signal sequence.

The expression vector according to one exemplary embodiment of the present invention may be an expression vector capable of expressing the protein in a host cell, and the expression vector may be in any form such as a plasmid vector, a viral vector, a cosmid vector, a phagemid vector, or an artificial human chromosome.

In another aspect of the present invention, there is provided a pharmaceutical composition for immunosuppression comprising the fusion protein as an active ingredient.

The pharmaceutical composition for immunosuppression may be used for the treatment of autoimmune diseases or for immunosuppression of patients who has received an organ transplant.

According to another aspect of the present invention, there is provided a pharmaceutical composition for the treatment of autoimmune diseases comprising the fusion protein as an active ingredient.

The pharmaceutical composition may further contain a known immunosuppressant component. These known immunosuppressant include a glucocorticoid, a cytostatic agent, an anti-CD20 antibody, an anti-CD3 antibody, an anti-IL-2 antibody, an immunophilin inhibitor, interferon β, opioid, TNFα binding protein, mycophenolate, fingolimod or myriocin. The glucocorticoid may be prednisone, dexamethasone, or hydrocortisone. The cytostatic agent may be nitrogen mustard, nitrosourea, platinum coordination complex, folic acid analogue, azathioprine, mercaptopurine, fluorouracil, methotrexate, dactinomycin, anthracycline, mitomycin C, bleomycin or mithramycin. The immunophilin inhibitor may be cyclosporin, tacrolimus, sirolimus, or everolimus.

In the pharmaceutical composition, the autoimmune disease may be type 1 diabetes, alopecia areata, anti-phospholipid antibody syndrome, rheumatoid arthritis, psoriasis or psoriatic arthritis, multiple sclerosis, systemic lupus erythematosus, inflammatory bowel disease, Addison's disease, Graves' disease, Sjogren's syndrome, Guillain-Barre syndrome, Hashimoto's thyroiditis, Myasthenia gravis, inflammatory myophathy, autoimmune vasculitis, autoimmune hepatitis, hemorrhagic anemia, idiopathic thrombocytopenic purpura, primary biliary cirrhosis, scleroderma, vitiligo, pernicious anemia, or chronic celiac disease.

The composition may contain a pharmaceutically acceptable carrier, and may further include a pharmaceutically acceptable adjuvant, excipient, or diluent in addition to the carrier.

As used herein, the term "pharmaceutically acceptable" refers to a composition which is physiologically acceptable and generally does not cause an allergic reaction, such as a gastrointestinal disorder and dizziness, or a similar reaction when administered to a human. Examples of the carrier, the excipient, and the diluent include lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. Moreover, a filler, an anti-aggregation agent, a lubricant, a wetting agent, a fragrance, an emulsifier, and an antiseptic agent may be further contained.

Furthermore, when the pharmaceutical composition according to one exemplary embodiment of the present invention is administered to a mammal, the pharmaceutical composition can be formulated using methods known in the art to allow rapid, sustained, or delayed release of the active ingredient. Examples of the formulation include powder, a granule, a tablet, an emulsion, a syrup, an aerosol, a soft or hard gelatin capsule, a sterile injectable solution, and a sterile powder form.

The composition according to one exemplary embodiment of the present invention can be administered by various routes such as oral administration and parenteral administration, for example, suppository, transdermal, intravenous, intraperitoneal, intramuscular, intralesional, nasal, or intravertebral administration, and can be administered using an implantation device for sustained release or continuous or repeated release. The administration can be performed once or several times a day within a desired range, and can be performed at an interval such as once a week, twice a week, and once a month, and the duration of administration is also not particularly limited.

The composition according to one exemplary embodiment of the present invention can be formulated in a suitable form with a pharmaceutically acceptable carrier that is commonly used. Examples of the pharmaceutically acceptable carrier include carriers for parenteral administration such as water, suitable oil, a saline solution, aqueous glucose, and glycol, and a stabilizer and a preservative may be further contained. Examples of the suitable stabilizer include antioxidants such as sodium hydrogen sulfite, sodium sulfite, or ascorbic acid. Examples of the suitable preservative include benzalkonium chloride, methyl-or propyl-paraben, and chlorobutanol. Moreover, the composition according to the present invention may suitably contain a suspending agent, a solubilizer, a stabilizer, an isotonic agent, a preservative, an adsorption inhibitor, a surfactant, a diluent, an excipient, a pH adjuster, a painless agent, a buffer agent, an antioxidant, or the like if necessary, depending on the administration method or the formulation. The pharmaceutically acceptable carriers and formulations suitable for the present invention, including those exemplified above, are described in detail in the literature [Remington's Pharmaceutical Sciences, latest edition].

The dosage of the composition to a patient depends on many factors including a height of the patient, a body surface area, an age, a specific compound administered, a gender, a time and a route of administration, general health, and other drugs administered simultaneously. The pharmaceutically active protein can be administered in an amount of 100 ng/body weight (kg) to 10 mg/body weight (kg), more preferably 1 to 500 µg/kg (body weight), and most preferably 5 to 50 µg/kg (body weight), but the dosage can be adjusted in consideration of the abovementioned factors.

In another aspect of the present invention, there is provided a method for treating a subject suffering from an autoimmune disease comprising administering a therapeutically effective amount of the fusion protein to the subject.

As used herein, the term "therapeutically effective amount" means an amount sufficient to treat a disease at a reasonable benefit/risk ratio applicable to medical treatment, and the effective dose level is the type and severity of the subject, Age, sex, activity of the drug, sensitivity to the drug, time of administration, route of administration and rate of excretion, duration of treatment, factors including concurrent drugs and other factors well known in the medical field. The therapeutically effective amount of the composition of the present invention may be 0.1 mg/kg to 1 g/kg, more preferably 1 mg/kg to 500 mg/kg, but the effective dosage may be adjusted appropriately according to the patient's the age, sex and condition.

### EXAMPLES

Hereinafter, the present invention will be described in more detail through Examples and Experimental Examples. However, the present invention is not limited to Examples and Experimental Examples described below, and may be implemented in various other forms, and Examples and Experimental Examples described below are provided to enable the disclosure of the present invention to be complete and to fully convey the scope of the invention to those skilled in the art to which the present invention belongs.

### Example 1: Preparation of chimeric anti-CD154 antibody

The present inventors prepared an anti-CD154 antibody in which Fc region of the mouse-derived anti-CD154 antibody described in WO2016/182335A1 was replaced with a human IgG1 Fc region, which is an FcγRIIα binding inhibitory mutant introduced into the heavy chain CH2 and CH3 domains of an antibody (C10) and designated the chimeric antibody as 'C10M' (FIG. 1).

### Experimental Example 1: Analysis of blood clot formation of chimeric anti-CD154 antibody

138 µg of the chimeric antibody C10M prepared as described above was intravenously injected into wild-type mice and transgenic mice transduced with the FcγRIIα gene, respectively and 10 minutes later, blood was collected from the orbit and placed in an EDTA tube to perform CBC-platelet analysis. And then the animals were sacrificed, the lungs were excised to prepare cryo-sections, and then tissue staining was performed to count the number of thrombus.

As a result, as shown in FIG. 2, although C10M of Example 1 significantly reduced thrombogenesis in FcγRIIα transgenic mice compared to C10 to which the conventional wild-type IgG1 Fc was applied, however C10M shows that the thrombogenesis in FcγRIIα transgenic mice compared to wild type mice is more than twice higher, indicating that thrombogenesis cannot be completely suppressed.

### Example 2: Preparation of fusion protein

From the results of Example 1, the present inventors designed a fusion protein in which the Fab part of the C10M antibody was linked to a modified Fc region that does not bind to FcγR (FIG. 3). Particularly, a modified Fc region was designed to be modified so as not to bind to FcγR by introducing a mixed form of CH2 and CH3 of IgD and IgG4 while applying a hinge part of IgG1 as it is.

For the expression of the fusion protein, a polynucleotide encoding a hybrid heavy chain in which the heavy chain portion (V_{H}-CH1, SEQ ID NO: 9) of the Fab of the C10M antibody is linked with a modified Fc region (SEQ ID NO: 6) was prepared, and inserted into the pBispecific expression vector (Genexine, Inc.). In addition, a polynucleotide encoding a light chain portion (V_{L}-C_{L}, SEQ ID NO: 2) of the Fab of the C10M antibody was inserted into the pBispecific expression vector so that it can be controlled under a dual promoter.

The expression vector prepared above was transiently expressed using Thermo Fisher's ExpiCHO kit. Specifically, after mixing the vector construct prepared as described above with ExpiCHO-S cell and ExpiFectamine reagent included in the kit and the mixture was incubated for 1 day in an incubator equipped with 8% CO₂ and 37°C conditions, and then the temperature was reduced to 32°C and cultured until the 7^{th} day.

Then, protein A capture purification was performed, and it was confirmed whether the candidate material was purified through PAGE analysis and Western blot analysis under non-reducing and reducing conditions, and formulation was performed with a formulation buffer in consideration of the pI value of the candidate material. The formulated material was quantified using Nano drop and final purity was confirmed using SEC-HPLC.

As a result, as shown in FIGs. 4A to 4C, it was confirmed that not only the light chain and the heavy chain were normally expressed, but also appeared as a single band under non-reducing conditions, indicating that a normal hetero-tetramer was formed. In addition, as a result of SEC-HPLC analysis, the purity was very high at 97.9%.

Accordingly, the present inventors designated the chimeric antibody produced from the transient expression result as "PG-400-1", and produced a stable cell line capable of stably producing it.

Specifically, in order to develop a stable cell line expressing the PG-400-1 protein, the gene construct prepared above was cloned into a pGP30 expression vector through a sub-vector cloning step. Thereafter, the expression vector was transfected into CHO DG44 (from Dr. Chasin, Columbia University, USA) cells using a Neon-transfection system. As a first screening process, 10% dFBS (Gibco, USA, 30067-334), MEMα (Gibco, 12561, USA, Cat No. 12561-049) and HT⁺ (Gibco, USA, 11067-030) medium without HT (5-hydroxytrypamine) was used to perform HT selection, and LDC (Limiting Dilution Cloning; 96 wells, 30 plates) were performed to obtain a final cell line. Fed-batch culture was performed on the finally obtained cell line in 400 ml of HyCell CHO cell culture medium, and Protein A purification was performed on the culture medium obtained on the 15^{th} day of culture to confirm the purified protein. The quantity and purity of the purified protein were analyzed through SDS-PAGE and SEC-HPLC.

As a result, as shown in FIG. 4D, it was confirmed that the chimeric antibody produced in the stable cell line was also normally expressed. In addition, as shown in FIG. 4E, the chimeric antibody was found to be purified to a very high purity of 99.97% as a result of SEC-HPLC analysis.

### Experimental Example 2: hCD154 binding affinity analysis

The present inventors analyzed the binding affinity of the chimeric antibody prepared in Example 2 to hCD154 through biolayer interferometry (BLI) analysis.

To this end, the present inventors first attached CD154 protein to 96-well plates using Dip and Read^{™} Amine Reactive 2^{nd} Generation (AR2G) Reagent Kit (forteBio, Cat No. 18-5092). Specifically, after dispensing 200 µl of DW in a 96-well plate, an amine biosensor included in the kit was inserted and hydrated for 10 minutes. Subsequently, after dispensing 200 µl of DW was additionally to the plate, EDC:NHS was mixed in a 1:1 ratio in a volume corresponding to 1/20 of the required sample, then diluted in DW, and 200 µl was dispensed into the 96-well plate. Subsequently, CD154 protein was diluted to 5 µg/ml in 10 mM acetate solution (pH 6.0), and 200 µl was dispensed into the 96-well plate. Then, 200 µl of 1 M ethanolamine was added to the 96-well plate, and the biosensor plate and the sample plate were inserted into Octet^{®} K2 BLI analyzer and signals were measured. After the measurement was completed, 200 µl of the 1x Kinetics buffer was added to the sample plate, and then a baseline was determined. Subsequently, the chimeric antibody (C10M) and chimeric hybrid antibody (PG-400) prepared in the above Examples 1 and 2, respectively, were diluted in 1x Kinetic buffer at various concentrations (200 pM to 12.5 nM), then dispensed to the sample plate at 200 µl and then BLI (Bio-layer interferometry) analysis was performed to measure binding affinity using Octet^{®} K2 BLI analyzer.

**Table 1**

| | | |
|---|---|---|
| Examples | 1 | 2 |
| Avg. K_{D} (nM) | < 1E-12 | < 1E-12 |
| Avg. Kₒₙ (1/Ms) | 509,500±8,890 | 807,000±4,250 |
| Avg. K_{dis} (1/s) | < 1E-07 | < 1E-07 |
| Avg. R² | 0.96 | 0.93 |

As a result, as shown in FIG. 5 and Table 1, it was confirmed that the chimeric antibody of the Example 1 of the present invention and the chimeric hybrid antibody of the Example 2 of the present invention, respectively, had sufficiently high affinity for CD154, although it was not possible to distinguish values of 1.0 pM or less, due to the limitations of the measuring device.

### Example 3: Preparation of humanized hybrid antibody

Although the present inventors have sufficiently improved the disadvantages of conventional human antibodies by introducing a modified Fc region that does not bind FcγR to the PG-400-1, the antigen-binding site is still a mouse-derived antibody and thus it may induce unnecessary immune response in the human body. Therefore, in order to produce a safer hybrid antibody, the present inventors designed a humanized antibody in which the rest of framework except for the antigenic determinant of Fab, which is an antigen-binding fragment of the antibody, the basis of the present invention was substituted with corresponding sequence of human antibody, and designated it as "PG-400-2" (Tables 2 and 3). In both PG-400-1 and PG-400-2, the C-terminus of the modified Fc has an amino acid sequence of SEQ ID NO: 6 terminated with lysine (K) because no other protein is fused to the C-terminus.

In addition, the present inventors designed a fusion protein by linking IL-10 protein, a cytokine having immunosuppressive activity to the C-terminus of the modified Fc region, with a linker peptide having an amino acid sequence of SEQ ID NO: 12, and this was referred to as "PG-410" (Table 2). In the case of PG-410, a deleted type (SEQ ID NO: 5) was used in which the lysine residue at the C-terminus of the Fc region was deleted due to the addition of the IL-10 protein to the C-terminus of the Fc region.

**Table 2**

| | | |
|---|---|---|
| Heavy chain construction of humanized hybrid antibodies (PG-400-2 and PG-410) | | |

| Components | amino acid sequence | SEQ ID NOs. |
|---|---|---|
| Signal Sequence | MGWSCIILFLVATATGVHS | 10 |
| anti-CD 154 heavy chain (V_{H}-CH1) | QVQLVQSGAEVVKPGASVKLSCKASGYIFTSYYMYWVK QAPGQGLEWIGEINPSNGDTNFNEKFKSKATLTVDKSAST AYMELSSLRSEDTAVYYCTRSDGRNDMDSWGQGTLVTV SSASTKGPSVFPLAPSSKSTSGGTAALGCLVKDYFPEPVTV SWNSGALTSGVHTFPAVLQSSGLYSLSSVVTVPSSSLGTQT YICNVNHKPSNTKVDKKV | 11 |
| hinge | EPKSCDKTHT CPPCP | 40 |
| modified Fc (PG-400-2) | SHTQPLGVFL FPPKPKDQLM ISRTPEVTCV VVDVSQEDPE VQFNWYVDGV EVHNAKTKPR EEQFNSTYRV VSVLTVLHQD WLNGKEYKCK VSNKGLPSSI EKTISKAKGQ PREPQVYTLP PSQEEMTKNQ VSLTCLVKGF YPSDIAVEWE SNGQPENNYK TTPPVLDSDG SFFLYSRLTV DKSRWQEGNV FSCSVLHEAL HNHYTQKSLS LSLG(K) | 5(6) |
| linker peptide (PG-410 only) | AAGSGGGGGS GGGGSGGGGS | 12 |
| IL-10Vm (PG-410 only) | SPGQGTQSENSCTHFPGNLPNMLRDLRDAFSRVKTFFQM KDQLDNLLLKESLLEDFKGYLGCQALSEMIQFYLEEVMP QAENQDPDAKAHVNSLGENLKTLRLRLRRCHRFLPCENG GSGGSGGSKSKAVEQVKNAFNKLQEKGIYKAMSEFDIFIN YIEAYMTMKIRN | 7 |

**Table 3**

| Light chain construction of humanized hybrid antibodies (PG-400-2 and PG-410) | | |
|---|---|---|
| Components | amino acid sequence | SEQ ID NOs. |
| Signal Sequence | METDTLLLWVLLLWVPGSTG | 13 |
| anti-CD 154 light chain (V_{L}-C_{L}) | DIVLTQSPATLSVSPGERATISCRASQRVSSSTYSYMHWY QQKPGQPPKLLIKYASNLESGVPARFSGSGSGTDFTLTISS VEPEDFATYYCQHSWEIPPTFGGGTKLEIKRTVAAPSVFIF PPSDEQLKSGTASVVCLLNNFYPREAKVQWKVDNALQS GNSQESVTEQDSKDSTYSLSSTLTLSKADYEKHKVYACE VTHQGLSSPVTKSFNRGEC | 4 |

### Example 4: Preparation of anti-CD154 scFv-modified Fc-IL-10 fusion protein

The present inventors selected the variable region portion of the Fab of the humanized anti-CD154 antibody and then designed an scFv using the Fab, and this was introduced into the fusion protein of Example 3 instead of the V_{H}-CH1 portion of the heavy chain construct of the anti-CD 154 antibody. By inserting into a single chain-based antibody-binding-fragment fusion protein was designed, it was designated as 'PG-420' (Table 4). Although the anti-CD 154 scFv is composed of a light chain variable region (V_{L})-linker-heavy chain variable region (V_{H}) in this embodiment, alternatives such as V_{H}-linker-V_{L} sequence may be used because it maintains binding affinity to CD154 and moreover it is possible to use various types of linkers.

**Table 4**

| Constitution of anti-CD 154 scFv-modified Fc-IL-10 fusion protein | | | |
|---|---|---|---|
| Components | | amino acid sequence | SEQ ID NOs. |
| Signal sequence | | METDTLLLWV LLLWVPGSTG | 13 |
| anti-CD154 scFv | V_{L} | DIVLTQSPATLSVSPGERATISCRASQRVSSSTYSYMHWYQQ KPGQPPKLLIKYASNLESGVPARFSGSGSGTDFTLTISSVEPE DFATYYCQHSWEIPPTFGGGTKLEIKRTVAAPSV | 14 |
| | linker 1 | GSTSGSGKPGSGEGS | 39 |
| | V_{H} | QVQLVQSGAEVVKPGASVKLSCKASGYIFTSYYMYWVKQ APGQGLEWIGEINPSNGDTNFNEKFKSKATLTVDKSASTAY MELSSLRSEDTAVYYCTRSDGRNDMDSWGQGTLVTVSS | 15 |
| hinge | | EPKSCDKTHT CPPCP or EPKSSDKTHT CPPCP | 40 or 41 |
| modified Fc | | SHTQPLGVFL FPPKPKDQLM ISRTPEVTCV VVDVSQEDPE VQFNWYVDGV EVHNAKTKPR EEQFNSTYRV VSVLTVLHQD WLNGKEYKCK VSNKGLPSSI EKTISKAKGQ PREPQVYTLP PSQEEMTKNQ VSLTCLVKGF YPSDIAVEWE SNGQPENNYK TTPPVLDSDG SFFLYSRLTV DKSRWQEGNV FSCSVLHEAL HNHYTQKSLS LSLG | 5 |
| linker 2 | | AAGSGGGGGS GGGGSGGGGS | 12 |
| IL-10Vm | | SPGQGTQSENSCTHFPGNLPNMLRDLRDAFSRVKTFFQMK DQLDNLLLKESLLEDFKGYLGCQALSEMIQFYLEEVMPQA ENQDPDAKAHVNSLGENLKTLRLRLRRCHRFLPCENGGSG GSGGSKSKAVEQVKNAFNKLQEKGIYKAMSEFDIFINYIEA YMTMKIRN | 7 |

### Example 5: Production of fusion protein sample

The fusion protein in Example 2 was not a humanized antibody, but a chimeric antibody, and it was produced in a small amount on a laboratory scale. In addition, since the host cells are CHO cells not derived from human cells, the sugar chain pattern of the produced protein may be different from that of the human protein. Accordingly, the present inventors tried to confirm whether the recombinant humanized antibodies (PG-400 and PG-410) of Example 3 were normally produced by changing the host cell to a human cell, HEK293 cell, and expanding the culture scale to 3 L.

### 5-1: Production of PG-400-2 protein

After preparing a polynucleotide encoding each of the heavy and light chains of the PG-400-2 protein designed in Example 4, and inserting it into the N293F Vector system (YBiologics, Korea) similarly to Example 2, and then the vector were cotransfected into HEK293 cells. The cotransfected HEK293 cells were cultured for 6 days at 3 L scale at 37° C, 5% CO ₂ through a bioreactor (FreeStyle 293 Expression Medium, Gibco), and then protein expression was confirmed by PAGE analysis under reducing conditions. As a result, it was confirmed that the protein was normally produced, as shown in FIG. 6A. Subsequently, the present inventors performed affinity chromatography by loading the cell culture solution on a column filled with Protein A bead. After washing with wash buffer (D-PBS, pH 7.4), 0.1 mM glycine buffer (pH 3.3) was added to elute the protein. Next, the protein was further purified through gel filtration using Superdex 200 column, running buffer (D-PBS, pH 7.4) and elution buffer (D-PBS, pH 7.4).

Protein production and purification were confirmed through PAGE analysis of the final purified protein under reduced and non-reducing conditions. As a result, it was confirmed that it was purified with high purity as shown in FIG 6B.

Next, the present inventors, first of all, put the purified protein into a pre-hydrated regenerated cellulose (RC) tube, then put it in a 2 L beaker containing a formulation buffer (PBS, pH 7.4) suitable for the pI value of the candidate material, and dialyzed at 4°C overnight to formulate the protein. The formulated material was quantified using Nano drop and the final purity was confirmed using SEC-HPLC. As a result, as shown in Table 5 and FIG. 6C, the total amount of protein was 184 mg and the productivity was 61 mg/L, the purity reached to 99.4%, indicating that it was purified with very high purity, and the endotoxin content was less than 0.38 EU/mg.

**Table 5**

| | | | | | | | | |
|---|---|---|---|---|---|---|---|---|
| Results of PG-400-2 test sample purification | | | | | | | | |

| material | host cell | volume | total amount of protein (mg) | yield (mg/L) | purity (%) | endotoxin contents (EU/mg) | purification process | formulation buffer |
|---|---|---|---|---|---|---|---|---|
| PG-400-2 | HEK293 | 3 L | 184 | 61 | 99.4 | < 0.38 | Protein A affinity chromatography gel filtration | PBS (pH7.4) |

Therefore, it was confirmed that the humanized anti-CD 154 antibody (PG-400-2) according to an embodiment of the present invention can be produced with high purity even though the culture scale is expanded in human cells.

### 5-2: Production of PG-410 protein

In addition, the present inventors prepared polynucleotides encoding each of the heavy and light chains of the PG-410 protein designed in Example 4, and then cloned them into the N293F Vector system (YBiologics, Korea) in the same manner as in Example 5-1. The vectors were cotransfected into HEK293 cells.

PG-410 protein according to an embodiment of the present invention was purified in the same manner as in Example 5-1, except that the cotransfected HEK293 cells were cultured to 2.3 L and the gel filtration step was omitted.

**Table 6**

| Results of PG-410 test sample purification | | | | | | | | |
|---|---|---|---|---|---|---|---|---|
| material | host cell | volume | total amount of protein (mg) | yield (mg/L) | purity (%) | endotoxin contents (EU/mg) | purification process | formulation buffer |
| PG-410 | HEK293 | 2.3 L | 295 | 128.2 | 92.6 | < 0.021 | Protein A affinity chromatography | PBS (pH7.4) |

As a result, as shown in Table 6 and FIGs. 7A to 7C, the IL-10-linked humanized anti-CD 154 antibody (PG-410) according to an embodiment of the present invention was purified with high purity even though additional protein (IL-10) was linked to the C-terminal and a gel filtration step was omitted. In particular, the protein yield was higher at 128.2 mg/L although the culture scale was smaller than that of PG-400-2 and the content of endotoxin was also very low at 0.021 EU/mg,.

### Experimental Example 3: Binding Affinity Analysis with CD154

The present inventors analyzed whether PG-400-2 and PG-410 prepared in Example 4 also specifically bind to CD154 like the fusion protein prepared in the Example 2 through Bio-layer interferometry (BLI) analysis.

Specifically, 200 µl of distilled water was dispensed into a flat-bottom 96-well black plate, and then a biosensor tray was placed on the black plate, and an amine biosensor was inserted and hydrated for 10 minutes. To determine the initial baseline, 200 µl of distilled water was dispensed into a new 96-well black sample plate. Subsequently, EDC:NHS was mixed in a 1:1 ratio in a volume corresponding to 1/20 of the required sample, then diluted in distilled water, and then 200 µl was dispensed into the hydrated 96-well black sample plate. A sample CD154 was diluted to 5 µg/ml in 10 mM acetic acid solution (pH 6.0), and 200 µl was dispensed into a 96-well black plate. Then, 200 µl of 1 M ethanolamine was added to the 96-well black plate, and the biosensor plate and the sample plate were inserted into the Octet^{®} K2 BLI analyzer and induced binding CD154 to the amine sensor measuring signals. After the measurement was completed, the 96-well black sample plate was removed from the device, and then 200 µl of 1x kinetic buffer was added to measure the baseline. Then, PG-400-2/PG-410, which is an antibody to be analyzed, was sequentially diluted in IX kinetic buffer so that the concentration was 200 to 12.5 nM, and then 200 µl of solution was dispensed to the 96-well black plate. Subsequently, the 96-well black plate was inserted into the Octet^{®} K2 BLI analysis device and the signal was measured while performing a reaction with CD154.

**Table 7**

| | | |
|---|---|---|
| Results of BLI analysis of PG-400-2 and PG-410 | | |

| Sample ID | PG-400-2 | PG-410 |
|---|---|---|
| K_{D} (nM) | 0.36 M | < 1E-12 |
| Kₒₙ (1/Ms) | 8.47E+05 | 1.96E+05 |
| K_{dis} (1/s) | 3.11E-04 | < 1E-07 |

Consequently, as shown in Table 7 and FIGs. 8A and 8B, both PG-400-2 and PG-410 were specifically bound to CD 154. In particular, in the case of PG-410, the Kₒₙ value was about 4.3 times lower than that of PG-400-2, but the K_{dis} value of PG-410 was < 1E-07, showing high affinity to CD154. This shows that PG-410 has a higher overall binding affinity (more than 3,000 times higher) than PG-400-2 due to its lower post-binding dissociation rate than PG-400-2, although the binding of PG-410 to CD154 occurs somewhat later than PG-400-2. However, overall, both PG-400-2 and PG-410 were found to bind specifically to CD 154, and in the case of PG-410, a lower dissociation rate after binding is expected to help sustain the effectiveness of IL-10 in target cells expressing CD154.

### Experimental Example 4: Evaluation of immunosuppressive activity by IL-10

The present inventors investigated the effect of monomeric IL-10 variant (IL-10Vm) on the immune response of immune cells in order to determine whether IL-10 of recombinant humanized antibody (PG-410) added to the C-terminus of the heavy chain functions properly.

Specifically, the supernatant of the T75 flask containing the cultured macrophage Raw264.7 cells was removed and washed once with PBS. After adding 3 ml of TrypLE^{™} Select Enzyme, the cells were incubated in a 37°C incubator for 3-5 minutes. Subsequently, 10 ml of a new medium was added to a T75 flask, the cell suspension separated from the plate was dispensed in a 50 mL centrifugation tube, and then the tube was centrifuged at 1,500 rpm for 5 minutes. Then, the supernatant was removed, and the centrifugation tube was weakly tapped several times by hand to suspension the cells. Subsequently, the cells were counted after mixing with 5 mL of medium. A medium was added and diluted so that the cell concentration was adjusted to 1×10⁵ cells/ml, and then 100 µl was dispensed on the 96-well flat bottom plate. After 10 hours, PG-410 sequentially diluted to have a final concentration of 1000 to 0.003 nM was treated on a 96-well flat bottom plate by 50 µl. After 20 minutes, 400 ng/ml of LPS was treated. It was then incubated in a 37°C incubator for 16 hours, and the supernatant was collected the next day, and the concentration of TNFα was measured according to the manufacturer's protocol using the TNFα ELISA kit (FIG. 9A). Meanwhile, recombinant IL-10 was used as a positive control.

Consequently, as confirmed in Table 8 and FIG. 9B, recombinant humanized antibodies according to an embodiment of the present invention had about 100 times lower immunosuppressive activity than recombinant IL-10.

**Table 8**

| The results of macrophage immunosuppressive activity analysis of PG-410 and IL-10 | | | | | | | | | |
|---|---|---|---|---|---|---|---|---|---|
| Treated Conc. (nM) | 0.0003 | 0.0013 | 0.006 | 0.032 | 0.16 | 0.8 | 4 | 20 | 100 |
| Inhibition rate of IL-10 (%) | 10% | 17% | 39% | 55% | 64% | 70% | 71% | 73% | 73% |
| Treated Conc. (nM) | 0.003 | 0.013 | 0.06 | 0.32 | 1.6 | 8 | 40 | 200 | 1,000 |
| Inhibition rate of PG-410 (%) | 8% | 13% | 24% | 29% | 38% | 45% | 56% | 59% | 58% |

### Experimental Example 5: Single-time toxicity evaluation

Conventional recombinant IL-10 has been reported to cause anemia symptoms as concentration of hemoglobin in blood is decreased and thrombocytopenia as concentration of platelet with increasing dose or repeated administration (Tilg et al., J. Immunol. 164(4): 2204-2209, 2002; Fedorak et al., Gastroendocrinol. 119(6): 1473-1482, 2000). Accordingly, the present inventors performed a toxicity test for the GLP application test according to the 'Non-Clinical Test Management Standard' of the Food and Drug Administration Notification No. 2017-183 (August 30, 2017) for PG-410 prepared in Example 4 above by requesting Biotoxtech (Korea).

Specifically, the single-dose toxicity test method conducted by Biotoxtech was carried out as follows. Toxicity tests were performed using 5-week-old female ICR mice, and 3 animals were reared per cage under the specified breeding conditions. The acclimatization period was set for 1 week, during which general symptoms were observed, and only healthy animals were used in the test, and food and drinking water were freely consumed. The weight of animals used in the experiment was measured so that the average weight of each group was equalized, and individual identification was marked on the tail of the animals, and an individual identification card was attached to the breeding box. As a test substance, PG-410 was administered intravenously to mice once with sterile physiological saline as a control, and a maximum of 300 mg/kg was administered based on the body weight immediately before administration, and the toxicity test was examined by autopsy.

As a result, no mortality, general symptoms, weight change, and gross autopsy findings were observed in relation to the administration of the test substance.

### Experimental Example 6: Graft versus Host Disease (GVHD) Related Animal Experiment

The present inventors investigated whether the recombinant humanized antibody PG-410 according to an embodiment of the present invention can be used for the treatment of graft versus host disease, which is a major side effect occurring during organ transplantation, through an animal model experiments.

For this purpose, 6-week-old male NSG mice were purchased from Orient Bio (Korea), and general symptoms were observed every day while undergoing acclimatization for 7 days in the animal room for this test. A review of the test report for pathogens provided by the animal supplier showed no factors that could affect the test. During the acclimatization period, the weights of animals determined to be healthy were ranked and randomly distributed so that the average weight of each group was evenly distributed. Then, the test substance was formulated at 5 mpk (100 µg/head), and administered intravenously to mice before and 28 days after the induction of GVHD using sterile physiological saline as a control. The day after administration of the test substance, 200 µl of human peripheral blood mononuclear cells (PBMC, 1×10 ⁷ cells per animal) were administered intravenously to 20 NSG mice. Before and after PBMC administration, body weight was measured twice a week. GVHD induction was assessed twice a week through the five medical symptoms of GVHD weight loss, activity, posture, fur texture, and skin integrity. As a control, a carrier was used.

As a result, as can be seen in FIGs. 10B and 10C, the group administered with PG-410 did not lose weight, unlike the negative control group, and the survival rate was remarkably higher than that of the negative control group.

It has been reported that the onset of GVHD after PBMC administration and the proportion of human CD45⁺ leukocytes differentiated from the administered PBMCs have a close relationship (Ehx et al., Front. Immunol. 9: 1943, 2018). Thus, blood was collected on the 7^{th}, 14^{th}, 21^{st}, 28^{th}, 35^{th}, and 42^{nd} day after PBMC administration from the negative control group and the PG-410 administration group, respectively and the human CD45⁺ cell population was analyzed by flow cytometry. Specifically, blood collected on the 7^{th} day (D7) after PBMC administration was dispensed into 96-well round bottom plates by 50 µl. The blood was washed with FACS buffer, and then centrifuged at 1500 rpm for 5 minutes. After discarding the supernatant, the formed cell pellet was suspended in 50 µl FACS buffer diluted with 0.5 µl aqua fluorescent reactive dye, and reacted at 4°C for 20 minutes. After washing with FACS buffer and discarding the supernatant, 50 µl of antibody mixture (anti-human CD45, anti-mouse CD45) was added to each well, and reacted at room temperature for 20 minutes. Then, it was washed twice with FACS buffer and analyzed using a flow cytometer. The assay was repeated equally on the 14^{th}, 21^{st}, 28^{th}, and 35^{th} after PBMC administration.

As a result, as shown in FIG. 11, it was confirmed that the human CD45 cell population, an important marker for the pathogenesis of GVHD, was decreased in the PG-410 administration group compared to the negative control group.

### Experimental Example 7: Analysis of binding affinity of the modified Fc region to various Fc gamma receptors

As described above, the modified Fc region protein used in an embodiment of the present invention is a variant designed to remove the binding ability to Fc gamma receptors. Accordingly, the present inventors attempted to investigate whether the modified Fc region protein according to an embodiment of the present invention does not actually bind to various Fc gamma receptors.

To this end, the present inventors investigated binding affinity of modified Fc region to various Fc gamma receptors using GLP-1E-Fc fusion protein (referred to as "PG-11") prepared by linking GLP-1 receptor agonist consisting of amino acid represented by SEQ ID NO: 49 to the N-terminus of the modified Fc region protein having the same amino acid sequence as the modified Fc region protein (SEQ ID NO: 5 or 6) which is applied to the anti-CD 154 antibody fusion protein prepared according to an embodiment of the present invention using a linker peptide having amino acid sequence represented by SEQ ID NO: 43. The binding affinity was analyzed through BLI analysis. At this time, rituximab, an IgG1 subclass recombinant antibody, was used as a control.

As a result, as shown in FIG. 12, rituximab, an IgG1-based antibody, specifically bound to FcyRI and FcγRIIIa, whereas, PG-11, a fusion protein containing NTIG according to an embodiment of the present invention did not specifically bind to these receptors.

Furthermore, the present inventors compared and analyzed the binding affinity of the GLP-1E-Fc fusion protein according to an embodiment of the present invention and rituximab with FcyRIIa, FcyRIIb and FcyRIIIb. As a result, as shown in FIGs. 13A to 13D, it was confirmed that rituximab specifically binds to the three Fc gamma receptors, whereas the fusion protein according to an embodiment of the present invention exhibits very weak binding. However, considering profile of the sensogram, it is estimated to be non-specific binding.

As described above, since the modified Fc region protein used in the present invention does not bind to various Fc gamma receptors, unwanted side effects of conventional antibody therapeutics such as ADCC or CDC can be minimized.

The present invention has been described with reference to the above-described embodiments and experimental examples, but these are merely exemplary, and those of ordinary skill in the art will appreciate that various modifications and equivalent other embodiments are possible therefrom. Therefore, the true technical protection scope of the present invention should be determined by the technical spirit of the appended claims.

## Claims

1. A fusion protein comprising Fab or scFv specifically binding to human CD154, and an Fc region of immunoglobulin, wherein the Fc region is a modified Fc region mutated so as not to bind to the Fc gamma receptor.

2. The fusion protein according to claim 1, wherein the Fab specifically binding to CD154 consists of:
a light chain (V_{L}-C_{L}) consisting of an amino acid represented by SEQ ID NO: 2 and a heavy chain V_{H}-CH1 fragment consisting of an amino acid represented by SEQ ID NO: 9; or
a light chain (V_{L}-C_{L}) consisting of the amino acid sequence represented by SEQ ID NO: 4 and a heavy chain V_{H}-CH1 fragment consisting of the amino acid sequence represented by SEQ ID NO: 11.

3. The fusion protein according to claim 1, wherein the scFv is prepared by linking a light chain variable domain (V_{L}) consisting of an amino acid sequence represented by SEQ ID NO: 14 and a heavy chain variable region (V_{H}) consisting of an amino acid sequence represented by SEQ ID NO: 15 with a linker peptide.

4. The fusion protein according to claim 1, wherein Fc gamma receptor is FcγRI, FcyRIIA, FcγRIIB1, FcγRIIB2, FcγRIIIA, and/or FcγRIIIB.

5. The fusion protein according to claim 1, wherein the Fab or scFv specifically binding to CD154, and the Fc region is linked by a hinge or linker peptide.

6. The fusion protein according to claim 1, wherein the fusion protein consists of:
Ig heavy chain peptide having amino acid sequence represented by SEQ ID NO: 1 and Ig light chain peptide having amino acid sequence represented by SEQ ID NO: 2; or
Ig heavy chain peptide having amino acid sequence represented by SEQ ID NO: 3 and Ig light chain peptide having amino acid sequence represented by SEQ ID NO: 4.

7. The fusion protein according to claim 1, wherein the Fc region is a modified Fc region having the amino acid sequence of SEQ ID NO: 5 or 6.

8. The fusion protein according to claim 1, wherein the Fc region is a modified Fc region in which the 18^{th} and 196^{th} amino acids are substituted with threonine (T) and methionine (M), respectively in the amino acid sequence of SEQ ID NOs: 5 or 6.

9. The fusion protein according to claim 1, wherein an IL-10 protein is added to the C-terminus of the Fc region.

10. The fusion protein according to claim 9, wherein the IL-10 protein is an IL-10 variant protein in which isoleucine, the 87^{th} amino acid of the mature protein, is substituted with alanine.

11. The fusion protein according to claim 10, wherein the IL-10 protein is a monomeric IL-10 variant protein in which a peptide having a length of 6 to 12 a.a. is inserted between asparagine, the 116^{th} amino acid and lysine, the 117^{th} amino acid.

12. A fusion protein comprising sequentially a) an antigen-binding fragment or an antibody analog of an antibody that specifically binds to CD154; b) a modified Fc region that has been mutated so as not to bind to the Fc gamma receptor; and c) an IL-10 protein.

13. The fusion protein according to claim 12, further comprising at least one linker peptide independently between a) and b) and/or b) and c).

14. The fusion protein according to claim 13, the linker peptide between a) and b) is a hinge derived from an antibody.

15. The fusion protein according to claim 12, wherein the antigen-binding fragment of the antibody is Fab, F(ab')₂, Fab', scFv, diabody, triabody, sdAb (single domain antibody), VNAR or VHH

16. The fusion protein according to claim 12, wherein the antibody analog is affibody, affilin, affimer, affitin, alphabody, anticalin, avimer, DARPin, Fynomer, Kunitz domain peptide, monobody, repebody, VLR, or nanoCLAMP.

17. The fusion protein according claim 12, wherein the Fc region is a modified Fc region having the amino acid sequence of SEQ ID NO: 5 or 6.

18. The fusion protein according to claim 12, wherein the Fc region is a modified Fc region in which the 18^{th} and 196^{th} amino acids are substituted with threonine (T) and methionine (M), respectively in the amino acid sequence of SEQ ID NOs: 5 or 6.

19. The fusion protein according to claim 12, wherein the IL-10 protein is an IL-10 variant protein in which isoleucine, the 87^{th} amino acid, based on the mature form of human IL-10 protein, is substituted with alanine.

20. The fusion protein according to claim 20, wherein the IL-10 protein is a monomeric IL-10 variant protein in which a linker (spacer) peptide having a length of 6 to 12 a.a. is inserted between asparagine, the 116^{th} amino acid and lysine, the 117^{th} amino acid, based on the mature form of the human IL-10.

21. A polynucleotide encoding the fusion protein of any one among claims 12 to 20.

22. A vector comprising the polynucleotide of claim 21.

23. A pharmaceutical composition for immunosuppression comprising the fusion protein of any one among claims 1 to 20 as an active ingredient.

24. The pharmaceutical composition according to claim 23, wherein the composition is used for immunosuppression of patients who has received an organ transplant.

25. A pharmaceutical composition for the treatment of autoimmune disease comprising the fusion protein of any one among claims 1 to 20 as an active ingredient.

26. The pharmaceutical composition according to claim 25, wherein the autoimmune disease is type 1 diabetes, alopecia areata, anti-phospholipid antibody syndrome, rheumatoid arthritis, psoriasis or psoriatic arthritis, multiple sclerosis, systemic lupus erythematosus, inflammatory bowel disease, Addison's disease, Graves' disease, Sjögren's syndrome, Guillain-Barre syndrome, Hashimoto's thyroiditis, Myasthenia gravis, inflammatory myophathy, autoimmune vasculitis, autoimmune hepatitis, hemorrhagic anemia, idiopathic thrombocytopenic purpura, primary biliary cirrhosis, scleroderma, vitiligo, pernicious anemia, or chronic celiac disease.
